(19) Europäisches Patentamt

European Patent Office

Office européen des brevets

(11) **EP 1 104 488 B1**

(12) **EUROPEAN PATENT SPECIFICATION**

(45) Date of publication and mention
of the grant of the patent:
**25.10.2006 Bulletin 2006/43**

(51) Int Cl.:
*C12Q 1/68* (2006.01)   *G01N 33/68* (2006.01)
*G06F 19/00* (2006.01)

(21) Application number: **99935746.0**

(22) Date of filing: **21.07.1999**

(86) International application number:
**PCT/US1999/016417**

(87) International publication number:
**WO 2000/005414 (03.02.2000 Gazette 2000/05)**

(54) **LINKING GENE SEQUENCE TO GENE FUNCTION BY THREE DIMENSIONAL (3D) PROTEIN STRUCTURE DETERMINATION**

VERBINDEN EINER GENSEQUENZ MIT EINER GENFUNKTION MITTELS DREIDIMENSIONALER (3D) PROTEINSTRUKTURBESTIMMUNG

ETABLISSEMENT DE LIEN ENTRE UNE SEQUENCE DE GENE ET UNE FONCTION DE GENE PAR DETERMINATION DE LA STRUCTURE DE PROTEINE EN TROIS DIMENSIONS (3D)

(84) Designated Contracting States:
**AT BE CH CY DE DK ES FI FR GB GR IE IT LI LU MC NL PT SE**

(30) Priority: **21.07.1998 US 93641 P**
**29.10.1998 US 181601**

(43) Date of publication of application:
**06.06.2001 Bulletin 2001/23**

(73) Proprietor: **Rutgers, The State University**
**Piscataway, NJ 08854-8010 (US)**

(72) Inventors:
• **ANDERSON, Stephen**
**Princeton, NJ 08540 (US)**
• **MONTELIONE, Gaetano**
**Highland Park, NJ 08904 (US)**
• **HUANG, Yuanpeng**
**Piscataway, NJ 08854-8010 (US)**

(74) Representative: **Suèr, Steven Johannes et al**
**Ablett & Stebbing,**
**Caparo House,**
**101-103 Baker Street**
**London W1M 1FD (GB)**

(56) References cited:
• **WALLACE A C ET AL: "TESS: A geometric hashing algorithm for deriving 3D coordinate templates for searching structural databases. Application to enzyme active sites" PROTEIN SCIENCE, vol. 6, 1997, pages 2308-2323, XP002204929 US**
• **MONTELIONE G T ET AL: "SEQUENCE-SPECIFIC PROTON NMR ASSIGNMENTS AND IDENTIFICATION OF TWO SMALL ANTIPARALLEL BETA-SHEETS IN THE SOLUTION STRUCTURE OF RECOMBINANT HUMAN TRANSFORMING GROWTH FACTORS ALPHA" PROCEEDINGS OF THE NATIONAL ACADEMY OF SCIENCES OF THE UNITED STATES, vol. 86, no. 5, 1989, pages 1519-1523, XP001120371 1989 ISSN: 0027-8424**
• **WÜTHRICH K: "NMR - THIS OTHER METHOD FOR PROTEIN AND NUCLEIC ACID STRUCTURE DETERMINATION" ACTA CRYSTALLOGRAPHICA SECTION D: BIOLOGICAL CRYSTALLOGRAPHY, MUNKSGAARD PUBLISHERS LTD. COPENHAGEN, DK, vol. 51, May 1995 (1995-05), pages 249-270, XP002916509 ISSN: 0907-4449**
• **ZIMMERMAN DIANE E ET AL: "Automated analysis of protein NMR assignments using methods from artificial intelligence." JOURNAL OF MOLECULAR BIOLOGY, vol. 269, no. 4, 1997, pages 592-610, XP002223143 ISSN: 0022-2836**

EP 1 104 488 B1

- NILGES MICHAEL: "Calculation of protein structures with ambiguous distance restraints: Automated assignment of ambiguous NOE crosspeaks and disulphide connectivities." JOURNAL OF MOLECULAR BIOLOGY, vol. 245, no. 5, 1995, pages 645-660, XP002223144 ISSN: 0022-2836
- FISCHER, DANIEL ET AL: "A 3D sequence-independent representation of the protein data bank" PROTEIN ENGINEERING (1995), 8(10), 981-97 , XP009001860
- WALLACE ET AL: 'Derivation of 3D Coordinate Templates for Searching Structural Databases: Application to Ser-His-Asp Catalytic Triads in the Serine Proteinases and Lipases' PROTEIN SCIENCE vol. 5, 1996, pages 1001 - 1013, XP002922934
- FRIEDRICHS ET AL: 'An Automated Procedure for the Assignment of Protein 1HN, 15N, 13Calpha, 13Cbeta and 1Hbeta Resonances' J. BIOMOL. NMR. vol. 4, 1994, pages 703 - 726, XP002922935
- FARBER ET AL: 'Determination of Eukaryotic Protein Coding Regions Using Neural Networks and Information Theory' J. MOL. BIOL., vol. 226, 1992, pages 471 - 479, XP002922936
- BAGBY ET AL: 'The Button Test: A Small Scale Method of using Microdialysis Cells for Assessing Protein Solubility at Concentrations Suitable for NMR' J. BIOMOL. NMR. vol. 10, 1997, pages 279 - 282, XP002922937
- ORENGO ET AL: 'CATH - a Hierarchic Classification of Protein Domain Structures' STRUCTURE vol. 5, no. 8, 1997, pages 1093 - 1108, XP002922938

**Description**

**FIELD OF THE INVENTION**

The present invention pertains to methods for elucidating the function of proteins and protein domains by examination of their three dimensional structure, and more specifically, to the use of bioinformatics, molecular biology, and nuclear magnetic resonance (NMR) tools to enable the rapid and automated determination of functions, as a means of genome analysis.

**BACKGROUND OF THE INVENTION**

One of the most powerful ways of identifying the biochemical and medical function of a gene product is to determine its three-dimensional structure. Although there are numerous examples in which the primary (i.e., linear) structure of a protein has provided key clues to its biochemical function, three dimensional (3D) structure determination is considered to be more definitive at establishing biochemical function. The process of elucidating the 3D structure of large molecules, such as proteins is generally thought of as slow and expensive.

In the past, most drugs were discovered by screening proprietary chemicals with animal models or receptor libraries. Today, this approach is being replaced by "combinatorial chemistry" and "rational drug design". These are the primary methods being used in the development of, for example, drugs targeted at the enzymes of the human AIDS virus.

What limits the drug discovery process today is not screening or medicinal chemistry but the rate that the approximately 100,000 proteins in the human body can be identified and prioritized as potential drug targets. Of particular significance for the pharmaceutical industry are the emerging disciplines of bioinformatics and functional genomics. Application of technologies developed in these areas will allow companies to identify, in the next decade, the bulk of the most significant new drug targets. It has been estimated that about 10.000 genes from the human genome are of potential value in human medicine, but only a few percent of these genes have been isolated so far. However, it is reported that by the year 2005 the raw sequence data for all of these genes will have been determined by the Human Genome Project (HGP).

**I. PROTEIN STRUCTURE**

It is a generally accepted principle of biology that a protein's primary sequence is the main determinant of its tertiary structure. Anfinsen, *Science 181*:223-230 (1973): Anfinsen and Scheraga, *Adv. Prot. Chem. 29*:205-300 (1975); and Baldwin, *Ann. Rev. Biochem. 44*:453-475 (1975). For over a decade, researchers have been studying the theoretical and practical aspects of the folding of recombinant proteins.

For example, the "genetics" of protein folding using mutants of bovine pancreatic trypsin inhibitor (BPTI) has been studied. Mutants of BPTI were prepared in which several cysteine residues were replaced by alanine or threonine residues. These mutants were then expressed in a heterologous *E. coli* expression system. Although these mutants were found to fold into the proper conformation, the rate of the mutant folding was somewhat slower than that exhibited by wild-type BPTI. Marks *et al., Science 325:*1370-1373 (1987).

Ma *et al.* have also studied the genetics of protein folding using mutants of BPTI. Ma *et al., Biochemistry 36*: 3728-3736 (1997). The model system described by Ma *et al.* predicts that a "rearrangement" mechanism to form buried disulfides at a late stage in the folding reaction may be a common feature of redox folding pathways for surface disulfide-containing proteins of high stability.

Nilsson *et al.* have reported that factors, such as peptidyl prolyl isomerase, protein disulfide isomerase, thioredoxin, and Sec B, may interact with the unfolded forms of specific classes of proteins, while members of the hsp70/DnaK and hsp60/GroEL molecular chaperone families may play a more general role in protein folding. Nilsson *et al.*, *Ann. Rev. Microbiol. 45*: 607-635 (1991). Nilsson *et al.* further disclose that intrinsic folding rates, or even translation rates, of nascent proteins may be optimized by natural selection. Secretion, proteolysis and aggregation are other in vivo processes that depend greatly in the folding behavior of a given protein. Thus, protein folding involves an interplay between the intrinsic biophysical properties of a protein, in both its folded and unfolded states, and various accessory proteins that aid in the process.

Proteins are generally composed of one or more autonomously-folding units known as domains, Kim *et al., Ann. Rev Biochem. 59*:631-660 (1990); Nilsson *et al., Ann. Rev. Microbiol. 45*:607-635 (1991). Multidomain proteins in higher organisms are encoded by genes containing multiple exons. Combinatorial shuffling of exons during evolution has produced novel proteins with different domain arrangements having different associated functions. This is thought to have greatly increased the ability of higher organisms to respond to environmental challenges because, via recombinational events, it has enabled genomes to readily add, subtract, or rearrange discrete functionalities within a given protein, Patthy, *Cell 41*:657-663 (1985); Patthy, *Curr. Opin. Struct. Bio. 4*:383-392 (1994): and Long *et al., Science 92*:

12495-12499 (1995).

## II. INTERPRETATION OF A PROTEIN STRUCTURE

Several methods have been used to elucidate the 3D structure of a given protein molecule. Chiefly, these methods are X-ray crystallography and Nuclear Magnetic Resonance (NMR).

### A. X-Ray Crystallography

X-ray crystallography is a technique that directly images molecules. A crystal of the molecule to be visualized is exposed to a collimated beam of monochromatic X-rays and the consequent diffraction pattern is recorded on a photographic film or by a radiation counter. The intensities of the diffraction maxima are then used to construct mathematically the three-dimensional image of the crystal structure. X-rays interact almost exclusively with the electrons in the matter and not the nuclei.

The spacing of atoms in a crystal lattice can be determined by measuring the angle and intensities at which a beam of X-rays of a given wave length is diffracted by the electron shells surrounding the atoms. Operationally, there are several steps in X-ray structural analysis. The amount of information obtained depends on the degree of structural order in the sample. Blundell *et al.* provide an advanced treatment of the principles of protein X-ray crystallography. Blundell *et al.*, *Protein Crystallography*, Academic Press (1976). Likewise, Wyckoff *et al.* provide a series of articles on the theory and practice of X-ray crystallography. Wyckoff *et al.* (Eds.), *Methods Enzymol. 114:* 330-386 (1985).

### B. Nuclear Magnetic Resonance (NMR)

The classical approach for the analysis of NMR resonance assignments was first outlined by Wüthrich. Wagner and co-workers. Wüthrich, "NMR of proteins and nucleic acids" Wiley. New York. New York (1986): Wüthrich, *Science 243*:45-50 (1989); Billeter *et al., J. Mol. Biol. 155*:321-346 (1982), all of which are herein incorporated by reference. For a general review of protein determination in solution by nuclear magnetic resonance spectroscopy, see Wüthrich, *Science 243*:45-50 (1989). See also. Billeter *et al., J. Mol. Biol. 155*:321-346 (1982).

Wüthrich's classical approach can be briefly summarized in the following seven steps:

Step 1: Identification of individual resonances associated with each spin system, and designation of key atom types (e.g., $H^N$, $H^\alpha$, N, $C^\alpha$, $C^\beta$, etc.)

Step 2: Classification of each identified spin system with respect to one or more possible amino acid residue type(s).

Step 3: Identification of possible sequential relations between spin systems using inter-residue NOESY or triple-resonance data.

Step 4: Unique mapping of strings of sequentially-connected spin systems to segments of the amino acid sequence, thus establishing "sequence specific assignments."

Step 5: Extension of assignments to resonances of peripheral side-chain nuclei in each spin system, and determination of stereospecific assignments.

Step 6: Generation of distance constraints using assigned resonance frequencies to interpret NOESY, scalar-coupling, and

*Proceedings of the First International Conference of Intelligent Systems for Molecular Biology.* Washington: AAAS Press (1994); Zimmerman *et al., J. Biomol. NMR 4*:241-256 (1994); Zimmerman *et al., Curr. Opin. Struct. Bio. 5*:664-673 (1995); and Zimmerman *et al., J. Mol. Bio. 269*:592-610 (1997).

Under traditional methodology, before a given protein is studied at the 3D level, the researcher had already obtained detailed experimental information regarding the protein's function and characteristics. The 3D structure is typically the last of many experiments performed over many years of study. The 3D structure information is then used to refine the researcher's understanding of the given protein. Thus, under traditional methodology, it is very rare that the 3D structure of a given protein is determined before its biochemical function has been determined by other methods.

The present invention represents a paradigm shift in methodology because the researcher would first determine the 3D structure of a protein of unknown function and then use this structure to gain clues as to its function, which would be subsequently validated by appropriate biochemical assays.

Wallace et al, Protein Science, 6, 2308-23 (1997) discloses an algorithm that creates 3D templates from 3D structure databases and allows the comparison of these templates with a new sequence so as to functionally characterize it. In particular, this document describes a method for determining whether a protein has a known 3D template comprised of three amino acids in a catalytic triad, which are brought into close proximity by the entire protein sequence.

## SUMMARY OF THE INVENTION

The present invention describes an integrated system for rapid determination of the three-dimensional structures of proteins and protein domains and application of this technology in a high-throughput analysis of human and other genomes for drug discovery purposes.

The "structure-function analysis engine" described herein has the potential to discover the functions of novel genes identified in the human and other genomes faster than existing genetic or purely computational bioinformatics methods.

The present.invention employs:

1. Bioinformatics methods, including the analysis of exon-exon phases and other methods for segmenting or "parsing" DNA sequences of novel genes into domain-encoding regions;

2. Robust and general "domain trapping" methods for producing correctly-folded recombinant protein domains of novel biomedically-important human disease gene products;

3. Robust and general methods for high level expression and isotopic enrichment of these domains for NMR and X-ray crystallographic studies;

4. Screening methods to identify protein domain constructs that exhibit the properties required for structural analysis by NMR or X-ray crystallography;

5. Computer software, NMR pulse sequences, and related NMR technologies that provide fully automated analysis of protein structures from NMR data;

6. NMR spectroscopy methods for determining 3D structures of these domains;

7. Improved methods for mapping new domain structures to proteins in the Protein Data Bank that have similar structures and biochemical functions;

8. A relational data base of the empirical properties of expressed domains for organizing and integrating the biophysical and biological information derived from these studies, as well as methods for making such relational data bases; and

9. A method for integrating all of the above into a large-scale, high-throughput macromolecular "structure-function analysis engine," and the application this "structure-function analysis engine" to the discovery of biochemical functions of hundreds of genes from humans and human pathogens.

The specific biomedical gene targets that this technology can be used to develop include:

1. Domains from the human Alzheimer's β peptide precursor protein (APP).

2. Domains from other proteins genetically implicated in neoplastic, metabolic, neurodegenerative, cardiovascular, psychiatric and inflammatory disorders.

3. Domains from proteins associated with infectious agents (e.g., bacteria, fungi and viruses).

The present invention provides a high-throughput method for determining a biochemical function of a protein or polypeptide domain of unknown function comprising: (A) identifying a putative polypeptide domain 50 to 300 amino acids in length that properly folds into a stable polypeptide domain, the stable polypeptide having a defined three dimensional structure; (B) determining three dimensional structure of the stable polypeptide domain from an automated analysis of NMR spectrometer spectra of said polypeptide domain, wherein said automated analysis is conducted by NOESY_Assign process; (C) comparing the determined three dimensional structure of the stable polypeptide domain to known three-dimensional structures in a protein data bank, wherein the comparison identifies known structures within the protein data bank that are homologous to the determined three dimensional structure; and (D) correlating a biochemical function corresponding to the identified homologous structure to a biochemical function for the stable polypeptide domain.

Further preferably, the method further comprises the prestep of parsing a target polynucleotide into at least one putative polypeptide domain.

Conveniently, said parsing is performed by a first computer algorithm, wherein said first computer algorithm is selected from a computer algorithm capable of determining exon phase boundaries of a polynucleotide, and a computer algorithm capable of determining interdomain boundaries encoded in a polynucleotide.

Preferably, the method further comprises use of a computer algorithm that compares the putative polypeptide domain sequence with known domain sequences stored within a database.

Further preferably, said NMR spectra are analyzed by a second computer algorithm that automatically assigns resonance assignments to the polypeptide sequence.

Preferably still, said identification of the said stable polypeptide domain comprises measuring a time course of amide hydrogen-deuterium exchange.

In preferred embodiments, prior to step (E), said stable polypeptide domain is optimally solubilized, said optimum solubilization comprising:

i) preparing an array of microdialysis buttons, wherein each of the said microdialysis buttons contains at least substantially 1 $\mu$l of a substantially 1mM solution of said stable polypeptide domain;
ii) dialyzing each member of said array of microdialysis buttons against a different dialysis buffer;
iii) analyzing each of said dialyzed microdialysis buttons to determine whether said stable polypeptide domain has remained soluble; and
iv) selecting the polypeptide domain having optimum solubility characteristics for NMR spectroscopy.

Preferably, said comparison of said determined three dimensional structure to said known three-dimensional structures in the protein data bank is performed by a third computer algorithm that is capable of determining 3D structure homology between said determined three dimensional structures and a member of said protein data bank.
Further preferably, said third computer algorithm is selected from DALI, CATH and VAST.
Preferably still, said protein data bank is Protein Data Base ("PDB").
According to a further aspect of the present invention, there is provided a high-throughput method for determining a biochemical function of a polypeptide of unknown function encoded by a target polynucleotide, comprising the steps:

(A) identifying at least one putative polypeptide domain encoding region of the target polynucleotide ("parsing");
(B) expressing said putative polypeptide domain;
(C) determining whether said expressed putative polypeptide domain forms a stable polypeptide domain having a defined three dimensional structure ("trapping"):
(D) determining the three dimensional structure of the stable polypeptide domain by an automated NOESY_Assign process;
(E) comparing the determined three dimensional structure of the stable polypeptide domain to known three-dimensional structures of a protein data bank to determine whether any such known structures are homologous to the determined structure; and
(F) correlating a biochemical function corresponding to the homologous structure to a biochemical function for the stable polypeptide domain.

## BRIEF DESCRIPTION OF THE FIGURES

Figure 1 provides a flow chart of a high-throughp structure/function analysis system.
Figure 2A provides the far UV circular dichroism spectra of the purified recombinant APP NTD2-3 domain. Figure 2B provides the near UV circular dichroism spectra of the purified recombinant APP NTD2-3 domain.
Figure 3 provides a NMR spectra of the purified recombinant APP NTD2-3.
Figure 4 provides a hydrogen-deuterium exchange time course for the purified recombinant APP NTD2-3.
Figure 5 provides the results of a cooperative thermal unfolding experiment of the purified recombinant APP NTD2-3.
Figure 6 provides the results of the NMR $^{15}$N-$^{1}$H heteronuclear single quantum coherence (HSQC) spectral analysis of the NTD2-3 domain collected on a Varian Unity 500 spectrometer.
Figure 7 provides the 2D $^{15}$N-$^{1}$H$^{N}$ HSQC spectrum of CspA at pH 6.0 and 30°C.
Figure 8A provides an illustration of information derived from triple resonance data sets used for establishing intraresidue and sequential correlations of spin systems.

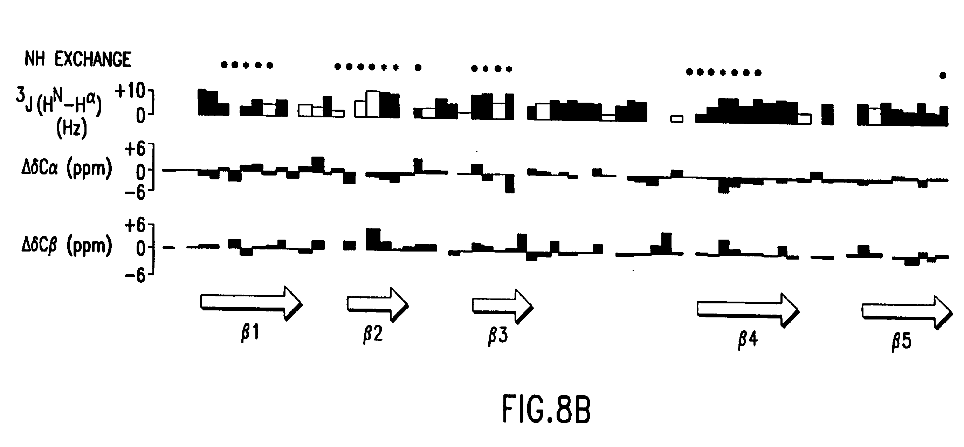

Figure 8B provides an illustration of NMR data used to identify structural elements in CspA. Slowly exchanging backbone amides ($t_{1/2}$ > 3 min at pH 6.0 and 30°C) are indicated by filled circles ($t_{1/2}$< 30 min) or starts ($t_{1/2}$> 30 min.). Values of $^{3}J$(H$^{N}$-H$^{\alpha}$) coupling constants are indicated by vertical bars; filled bars indicate that the data provided a useful estimate ($\pm$0.5Hz) of the corresponding coupling constant, while open bars indicate that the experimental data provide only an upper bound on its value. Values of conformation-dependent secondary shifts $\Delta\delta C^{\alpha}$ and $\Delta\delta C^{\beta}$ are plotted with solid bars. The locations of the five $\beta$-strands are indicated with arrows.
Figure 9 provides a flow chart of a NOESY_ASSIGN Process.
Figures 10A and B provide the 3D structure of the Zdom protein.
Figures 11, 12 and 13 provide results of an automated assignment analysis for the Zdom protein.
Figures 14, 15 and 16 provide results of a manual assignment analysis for the Zdom protein.
Figure 17 provides the 3D structure of the Cspa protein.
Figures 18, 19 and 20 provide results of an automated assignment analysis for the Cspa protein.
Figures 21, 22, and 23 provide results of a manual assignment analysis for the Cspa protein.

One of the best clues to a protein's function is its structure. Described herein is a structure-based bioinformatics platform to be used in "functional genomics" analyses of the torrent of DNA sequence data emerging from the international HGP. This technology will allow for the isolation of novel biopharmaceuticals and/or drug targets from gene sequence information with an efficiency that is far beyond present day capabilities. By developing extremely fast yet rigorous technologies for macromolecular structure determination, it is possible to convert the stream of one-dimensional DNA sequence information emerging from human genome research efforts into 3D protein structures. This 3D structural information can then be used to map these human gene products to protein families with similar biochemical functions.

Described herein is a "drug discovery search engine" that allows human genetic and genomic data to be smoothly interfaced with proven rational drug design and combinatorial chemistry approaches. The technology described herein enables determination of the structures for virtually the entire complement of human protein domains, encoded in the approximately 100,000 human genes.

## I. STRUCTURE SUGGESTS FUNCTION

It is a tenet of modem structural biology that structure suggests function: a given protein "fold" tends to be used over and over again in nature for a restricted set of biological functions. Knowledge of the structure of a new protein often reveals kinship to a family of other proteins with already known functions, and thus provides strong clues regarding the biochemical function of the protein at hand. Holm *et al., Science 273*:595-603 (1996); Bork *et al., Curr. Opin. Struct. Bio. 4*:393-403 (1994); Brenner *et al., Proc. Natl. Acad Sci. (U.S.A). 95*:6073-6078 (1998).

This kinship relationship is a natural manifestation of the fact that families of protein molecules have evolved from a common ancestral molecule, and that in the course of this evolution the 3D structure is largely preserved while new, though chemically related, biochemical functions are adopted. This is precisely the reasoning behind the assigning of "expressed sequence tag" (EST) sequences to known protein families using one-dimensional sequence comparisons.

Evolution generally acts to conserve 3D structures rather than the amino acid sequences of proteins. For this reason, proteins have often evolved over time so that their sequences exhibit no obvious similarity while their structures remain highly homologous. In practical terms, this means that simple sequence comparisons overlook many -- and perhaps even most - instances of protein-protein relatedness. However, this relatedness, with all of its functional implications, can easily be identified by 3D structure comparisons.

The multidomain nature of many mammalian proteins makes them more difficult to express in recombinant form and also impedes their structure determination by X-ray crystallography or NMR. The expression and structure determination of an isolated domain is, in contrast, less problematical. Since an isolated domain comprises one or more discrete functional units in a protein, knowing structure-function information about a given individual domain in a multicomponent protein generally provides key information that can be used to proceed with drug development on the full-length protein. The "domain trapping" methods generate many novel gene products suitable for structural analysis by NMR spectroscopy and X-ray crystallography.

Recent developments in the areas of high-level protein expression technology, X-ray crystalography, heteronuclear NMR spectroscopy, and artificial intelligence (AI)-based structural analysis software, have dramatically improved the speed and lowered the cost of protein structure determination. Estimates of the total number of human genes in the genome (approximately $10^5$) contrast dramatically with estimates of the total number of protein folds in nature (approximately $10^3$), and it has been estimated that one-third to one-half of these folds have already been described. Chothia *et al., Nature 357*:543-544 (1992). Simple statistics imply that many new gene products will exhibit structures that map to existing fold classes associated with proteins of known biochemical function. Thus, the harvest of functional information about new human genes from this approach will be immediate.

## II. DESIGN OF A HIGH-THROUGHPUT SYSTEM FOR DETERMINING PROTEIN STRUCTURES AND FUNCTIONS

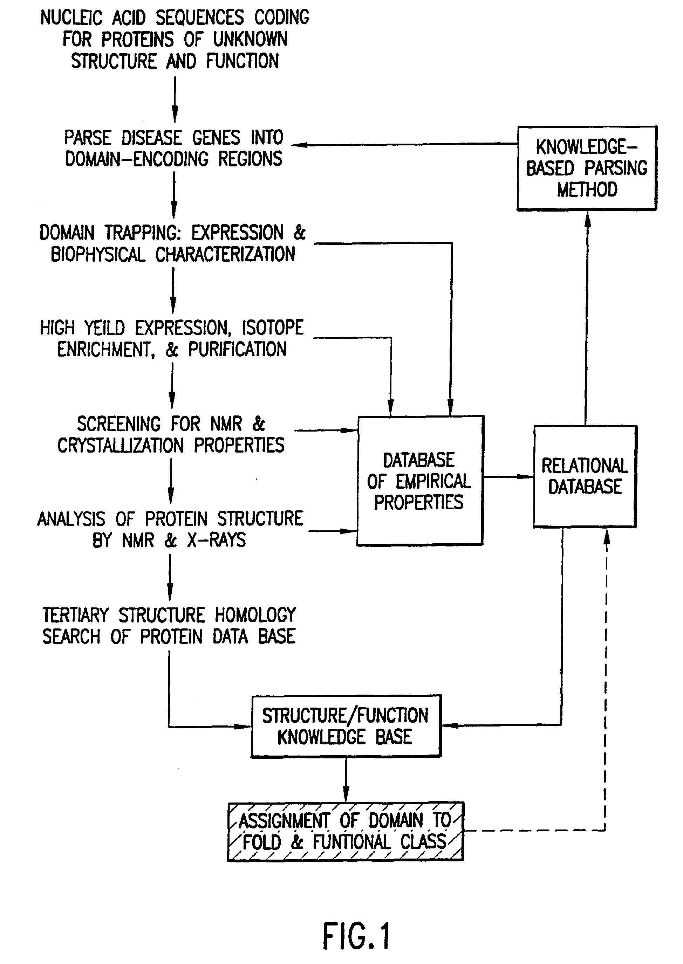

Figure 1 provides a flow chart of a high-throughput structure/function analysis used for analyzing human and pathogen gene products. This flow chart outlines the general methods described herein. Each sub-step is outlined in detail below. It is to be understood that the hardware disclosed herein can be or is operatively linked to one or more computers.

### A. Approaches For Identifying Novel Protein Domains

The present invention provides a method for predicting the location of domains and domain boundaries within a given DNA sequence. Under one method, this is accomplished through a knowledge based application which segments or "parses" genomic or cDNA sequences of genes into domain encoding sequences. Under another method, the knowledge based application can also segment or "parse" mRNA sequences into domain encoding sequences. Preferably, the knowledge based application is encoded within a computer algorithm software application. Preferably, this expert system applies rules developed on a set of experimentally-verified DNA sequence/protein domain comparisons that have been compiled from public sequence and protein structure databases. Thus, for a novel gene sequence, this expert system generates the predicted domains and/or domain boundaries which are then used to create domain-specific expression constructs.

Under another method, the gene sequence is parsed by the exon phase rule. Exon termini (5'- or 3') that begin or end within protein coding regions can be classified according to their "phase": an exon terminus that falls between two codons is called a "phase 0" terminus; an exon terminus that starts or stops after the first nucleotide in the codon is called a "phase 1" terminus; and an exon terminus that starts or stops after the second nucleotide in the codon is called a "phase 2" terminus. For example, where ("*") marks the positions of an exon-exon junction--

Phase 0:     *

5' ... -A-T-G-G-G-A-C-T-C- ... 3'

... - Met - Gly - Leu - ...

Phase 1:       *

5' ... -A-T-G-G-G-A-C-T-C- ... 3'

... - Met - Gly - Leu - ...

Phase 2:       *

5' ... -A-T-G-G-G-A-C-T-C- ... 3'

... - Met - Gly - Leu - ...

The genetic coding sequences for protein domains, which have been reported to have been "shuffled" between various genes during evolution, should be bounded by exon termini of the same phase (or by the N- or C-terminal ends of the holoprotein), otherwise insertion of these domains into a host gene would result in a frame-shift mutation in the downstream sequences upon splicing (Patthy, *Cell 41*:657-663 (1985); Patthy, *FEBS Letters 214*:1-7 (1987); Patthy, *Cur. Opin. Struct. Bio. 4*:383-392 (1994),

Therefore, the domain encoding regions should be bounded on both sides by phase 0 exon termini, by phase 1 exon termini, or by phase 2 exon termini, but not by termini of different phases.

As part of the mechanism of molecular evolution, structural and functional domains are mixed and matched between protein sequences through the processes of gene duplication and crossover. Accordingly, under one method domains are identified by looking for segments of gene sequences that are conserved across many genes from different organisms. Known domain families generally involve 50 - 300 amino-acid long segments that are observed as portions of many different proteins. Bioinformatics algorithms capable of identifying these conserved segments, or gene-fragment clusters, in the data base of gene sequences have been reported. These algorithms can be used to identify candidate domain-encoding regions in novel gene sequences. Gouzey *et al., Trends Biochem. Sci. 21*:493 (1994),

Under a second method, domains from gene sequence data are identified through predictions of their interdomain boundaries. There is ample evidence from molecular evolution and cell biology studies that information regarding domain boundaries is embedded in the sequences of protein coding genes. Some reports have claimed that rare codon clusters, which cause ribosomal pausing during translation, are correlated with domain boundaries. Purvis *et al., J. Mol. Biol. 193*:413-417 (1987); Nilsson *et al., Ann. Rev. Microbiol. 45*:607-635 (1991); Thanaraj *et al., Protein Sci. 5*:1973-1983 (1996); Thanaraj *et al., Protein Sci. 5*:1594-1612 (1996); and Guisez *et al., J. Theor. Biol. 162*:243-252 (1993). Messenger RNA secondary structure have also been reported to play such a "punctuation" role during translation.

One method employs an algorithm that identifies such sequence features and compares these data with the actual domain sequences in a relational database. The relational database contains domain sequence information of known and determined protein domains. It is understood that the relational database will expand over time such that each polypeptide domain determined using these methods will be added to the relational database. Under this method, it is possible to rigorously assess the reliability of these bioinformatics methods of domain prediction and, iteratively, modify the software to improve its reliability. Neural nets and genetic algorithms both can be used for deriving rules for domain boundaries from this knowledge base. This method markedly accelerates productivity by greatly reducing the number of expression constructs that would have to be tested in order to correctly parse a novel gene sequence into its component domain sequences.

Under another method, the solution structure of a protein or protein domain can be analyzed by a method that combines enzymatic proteolysis and matrix assisted laser desorption ionization mass spectrometry (Cohen *et al.*, *Protein Sci. 4*:1088-1099 (1995), Seielstad *et al.*, *Biochem. 34*:12605-12615 (1995).

This method is capable of inferring structural information from determinations of protection against enzymatic proteolysis as governed by solvent accessibility and protein flexibility. Preferably, the proteolytic enzymes employed by this method include trypsin, chymotrypsin, thermolysin, and ASP-N endoprotease.

## B. "Domain Trapping": Expression And Biophysical Characterization Of Putative Recombinant Protein Domains

With respect to genes of unknown function, the investigator, generally, does not have available an enzyme assay or other obvious activity-based means to assess the biochemical activity of a novel recombinant protein domain. The methods described herein have addressed this difficulty in a three-pronged manner. First they use a reliable and high yield expression system for protein expression. For example, a secretion-based protein A fusion system that is one of the most tested and reliable methods known for producing correctly-folded recombinant proteins in the *E. coli* periplasm. Nilsson *et al., Methods Enzymol. 185*:144-161 (1990),

Alternatively, the pET plasmid expression system may be used. Studier *et al., J. Mol. Bio. 189*:113-130 (1986). Second they, use a set of activity-independent biophysical criteria to assess whether the protein domain has properly folded. This set of criteria has been developed through extensive study of recombinantly-expressed protein folding mutants. Finally, based on the supposition that autonomous folding of the protein domain can be prevented due to too much or too little polypeptide sequence information, respectively, (Kim *et al., Ann. Rev. Biochem. 59*:631-660 (1990); Nilsson *et al., Ann. Rev. Microbiol. 45*:607-635 (1991), they use systematic strategies for identifying and trapping domains that enables it to use a combination of molecular biological and biophysical methods to experimentally parse any gene into its component domains. In other words, a polypeptide domain has a "defined three dimensional structure" when that polypeptide domain exhibits the activity-independent biophysical criteria of a properly folded domain.

Under one method, an activity-independent biophysical criteria used to assess the correctness of folding of a protein includes circular dichroism measurements. More preferably, characterization of an isolated domain of a protein is analyzed by circular dichroism measurements in the far UV. An ellipticity minimum at 222 nm is indicative of $\alpha$-helical secondary structure. Preferably, CD measurements at longer wavelengths are also determined (for a general review of CD and other methods, see Creighton, *Proteins: Structure and molecular properties,* 2nd Ed., W. H. Freeman & Co., New York, New York (1993, and related texts),

A signal in the aromatic region around 280 nm is consistent with the presence of Trp, Tyr, and Phe chromophores in an ordered environment, such as would be expected in the hydrophobic core of a folded protein. In general, assays for the affinity-purified expressed proteins that employ solely biophysical criteria have been designed based upon experience with the behavior of misfolded recombinant proteins.

It is preferable to further characterize the isolated domain by $^1$H-NMR spectroscopy. Preferably, the isolated domain is in a moderately concentrated solution (~100 $\mu$M). A high dispersion pattern of the proton resonance spectrum is reported to be characteristic of a well-folded polypeptide.

A time-course of amide hydrogen-deuterium exchange measurements can also be performed on the isolated domain. From this, it is possible to observe whether backbone NH groups are significantly protected within the domain. Significant protection is an indication that the hydrogen-bonded secondary structure is stabilized by tertiary interactions, which is consistent with a well-folded domain structure.

Finally, thermal denaturation experiments, monitored by intrinsic tryptophan fluorescence, can also be performed. These experiments are also capable of determining whether the isolated domain is a compact domain structure.

In principle, this is a general strategy. Thus, it can be used to parse many genes in the human genome that encode proteins of unknown biochemical function into their component domains and express correctly-folded polypeptide for structure/function studies. This general strategy can be easily modified to provide a high-throughput method for validating candidate domains identified by the bioinformatics methods of the present invention. For a typical 10 - 30 kD protein domain, 500 or 600 MHz one-dimensional (1D) NMR spectra can be obtained in tens of minutes using only small quantities (~ 200 $\mu$g) of protein. Using a continuous flow NMR probe with a microcomputer-controlled chromatography pump and simple sample changer, it is possible to automatically screen 50 - 100 candidate domains per day for folded structure. Those candidate domains which exhibit chemical shift dispersion indicative of ordered domain structure can then be further validated using the other biophysical techniques described above. An NMR spectrometer suitable for use is a Varian Unity 500 spectrometer.

## C. High Level Expression And Isotopic Enrichment

Uniform biosynthetic enrichment with $^{15}$N, $^{13}$C and $^{2}$H isotopes has been reported to be a prerequisite for the analysis of macromolecular structures by NMR spectroscopy. Some NMR strategies have also been reported to benefit from random enrichment with $^{2}$H isotopes. The principal obstacle for isotope-enriched protein production in most recombinant production systems is the high cost of the enriched media components (e.g. $^{13}$C-glucose @ $330/g), and the limiting possibilities for scale-up to controlled multi-liter fermenters. The less well-controlled conditions of shaker flask cultivations often result in lower protein production levels. The production of $^{15}$N-, $^{13}$C-, and/or $^{2}$H-enriched proteins thus requires an efficient system cable of providing high level production of the desired protein in small-scale bioreactors.

One method employs a bacterial production system for $^{15}$N, $^{13}$C-enriched recombinant proteins. Preferably, the bacterial production system is based on intracellular production of recombinant proteins in *E. coli* as fusions to an IgG-binding domain analogue, Z, derived from staphylococcal Protein A (Nilsson *et al.*, *Protein Eng. 1*:107-113 (1987); Altman *et al.*, *Protein Eng. 4*:593-600 (1991)). In this system, transcription is initiated from the efficient promoter of the *E. coli trp* operon. This allows for efficient intracellular production of fusion proteins. These fusion proteins can then be purified by IgG affinity chromatography. Using this approach it is possible to achieve high-level (40 - 200 mg/L) production in defined minimal media of a number of isotope-enriched proteins (see, for example, Jansson *et al.*, *J. Biomol. NMR 7*:131-141 (1996)).

Under another method, the recombinant isotope-enriched domain protein may be produced using pET plasmid expression vectors (Studier *et al., J. Mol. Biol.189*:113-130 (1986), herein incorporated by reference) under the control of the T7 RNA polymerase promoter (see, for example, Newkirk *et al., Proc. Nat'l Acad Sci. (U.S.A.) 91*:5114-5118 (1994); Chaterjee *et al., J. Biochem. 114*:663-669 (1993); and Shimotakahara *et al., Biochemistry 36*:6915-6929 (1997),

Under another method, $^{15}$N, $^{13}$C, $^{2}$H-enriched recombinant proteins can be produced by acclimating a bacterial production system to grow in 95% $^{2}H_2O$. Recombinant bacterial production hosts [e.g., the BL21 (DE3) strain] can be acclimated to grow in 95% $^{2}H_2O$ by successive passages in media containing increasing amounts of $^{2}H_2O$; protein production levels of acclimated bacteria grown in 95% $^{2}H_2O$ are identical to those obtained in $H_2O$. Using protiated [uniformly $^{13}$C-enriched]-glucose as the carbon source, $^{2}$H-enrichment levels of 70 - 80% can be achieved; high incorporation of $^{2}$H from the $^{2}H_2O$ solvent results from metabolic shuffling during amino acid biosynthesis. While the resulting proteins are not 100% perdeuterated, they are sufficiently enriched for the purpose of slowing $^{13}$C transverse relaxation rates and enhancing the sensitivity for certain types of triple-resonance NMR experiments. 100% perdeuterated samples can also be produced using $^{2}H_2O$ solvent and [uniformly $^{2}$H, $^{13}$C-enriched]-glucose as the carbon source.

Under one method, such isotope enriched proteins can be renatured by the method of Kim *et al.* which employs *in situ* refolding of proteins immobilized on a solid support. Kim *et al., Prot. Eng. 10*:445-462 (1997).

The isotope enriched proteins can also be renatured by the method of Maeda *et al.* which employs programmed reverse denaturant gradients. Maeda *et al., Protein Eng. 9*:95-100 (1996); Maeda *et al., Protein Eng. 9*:461-465 (1996). Under another method, the method of Kim *et al.* is coupled with the method of Maeda *et al.* Under yet another method, "active" folding agents, such as the molecular chaperones GroEL/ES, dnaK, dnaJ, etc., may be used to assist in protein folding. Nilsson *et al., Ann. Rev. Microbiol. 45*:607-635 (1991).

Preferably, the fusion vectors are constructed to interface with downstream refolding operations. Such vectors permit, for example, the binding of fusions to a solid support even under harshly denaturing conditions, such as high concentrations of guanidine hydrochloride and dithiothreitol. For such purposes, the preferred class of vector employs protein-RNA fusions. Such fusion proteins can be purified using oligonucleotide affinity columns with high specificity in the presence of chaotropic agents and strongly reducing conditions.

Under another method, other, non-bacterial, microbial systems, e.g., *Pichia*-based expression systems are employed. Kocken *et al., Anal. Biochem. 239*:111-112 (1996); Munshi *et al., Protein Expr. Purif. 11:*104-110 (1997); Laroche *et al., Bio/Technology 12*:1119-1124 (1994) Cregg *et al., Bio/Technology 11*:905-910 (1993),

Once the protein domain of interest has been expressed at high levels, it is necessary to purify large quantities of the protein domain for subsequent characterization. Preferably, at least 5-10 mg of the protein domain of interests is purified. More preferably, at least 50 mg of the protein domain of interest is purified.

Methods for preparing large quantities of a given protein of sufficient purity for domain structure modeling are generally known to those of skill in the art. Although not all methods for protein purification are applicable to a given protein of interest, it is generally understood that the following methods represent preferred embodiments: affinity chromatography, ammonium sulfate precipitation, dialysis, FPLC chromatography, ion exchange chromatography, ultracentrifugation, etc. For a general review of protein purification methodologies, see Burgess, *Protein Purification,* In: Oxender *et al.* (Eds.), *Protein Engineering,* pp. 71-82, Liss (1987); Jakoby, (Ed.), *Methods Enzymol. 104:* Part C (1984); Scopes, *Protein Purification: Principles and practice* (2nd ed.), Springer-Verlag (1987),

## D. Rapid Screening Of NMR And Crystallization Properties

One common problem for both NMR analysis and crystallization is poor solubility and/or slow precipitation of the protein sample. These properties are highly dependent on the pH, ionic strength, reducing agent concentration, and other properties of the buffer solvent. Thus, it is preferable to optimize these conditions to maximize solubility for NMR analysis and to optimize the conditions for protein crystallization.

Under one method, the optimization experiments are conducted with an array of microdialysis buttons to rapidly scan a plurality of standardized buffer conditions to identify those most suitable for NMR studies and/or crystallization of each domain construct (Bagby, *J. Biomol. NMR 10*:279-282 (1997). Preferably, each microdialysis button contains at least 1 μL of a ~1 mM protein solution. More preferably, each microdialysis button contains at least 5 μL of a ~1 mM protein solution. The microdialysis buttons are commercially available. Preferably, each microdialysis button is dialyzed against about 50 ml of dialysis buffer, such as in a 50 ml conical tube (Falcon). Preferably, the dialysis is performed at 4°C. However, the dialysis can be performed at temperatures ranging from 4°-40°C. Because NMR studies are routinely performed at room temperature for extended lengths of time, it is preferable that the protein remain in solution under these conditions.

Preferably, the protein samples are initially prepared in buffers containing 50% glycerol (which is not suitable for NMR studies but generally provides good solubility) and then dialyzed against different buffers containing little or no glycerol. With respect to NMR and X-ray crystallography studies, it is understood that a person of skill in the art would know what buffers could be used to prepare the protein for study. The skilled artisan typically has a set of 50-100 standard buffers which are used to prepare protein samples for subsequent studies. These buffers can then be modified if necessary to optimize the protein preparation. The ability of a given protein to remain soluble at high concentration or form suitable crystals is dependent on the pH of the solution, as well as the concentration of different salts, buffers, reagents, and temperature. Thus, the "button test" represents a preferred method because it facilitates the rapid screening of a multitude of conditions.

This "button test" analysis typically requires 5 - 10 mg of protein sample and can be completed in a few days. Preferably, multiple samples are analyzed in parallel. Preferably, the protein samples are analyzed under a dissecting microscope to determine whether the protein has remained in solution or whether the protein has aggregated. Using the "button test", a single technician could score solubility properties in 100 different buffers for -20 domains per week. Under another method, these screens can be carried out using state of the art laboratory automation technology.

Alternatively, the protein domain of interest is lyophilized and then resuspended in an appropriate buffer.

Having identified the conditions under which the protein domain of interest is soluble, dynamic light scattering can be used to examine its dispersive properties and aggregation tendency in different buffer conditions. Ferré-D'Amaré *et al., Structure 15*:357-359 (1994), Alternatively, Trp or Tyr fluorescence anisotropy can be used to measure rotational diffusion which is another measure of aggregation.

The "domain trapping" approach includes an evaluation of NMR properties, and all of the protein samples which pass this stage of the process will already meet basic spectroscopic quality criteria. Standard criteria used to determine the basic spectroscopic quality of a given protein, which are known to those of skill in the art, include a good dispersion pattern and a narrow peak width, etc.

Preferably, gel filtration chromatography and dynamic light scattering data are collected during the course of domain purification. Such data provide information about the oligomerization state of the domain being studied.

For domains of the appropriate size (< -30 kD), isotopically enriched samples are scored in terms of their suitability for structure determination by NMR using standard 2D HSQC, 2D NOESY, and/or 2D CBCANH triple-resonance spectra. The protein samples that provide good quality data for these NMR experiments are expected to provide good data in the full set of experiments required for automated structure determination. For each $^{15}$N, $^{13}$C enriched domain, this evaluation typically requires at least 5 - 10 mg of sample, and approximately 6 hours of NMR data collection. Preferably, the evaluation is performed on about 10 mg of sample. Thus, ~20 domains can be evaluated per "spectrometer-week" using the methods of the present invention. A "spectrometer-week", as used herein, means one skilled technician, working on one NMR machine would be able to evaluate approximately 20 domains in a given week.

Preferably, domains for structure determination by NMR are selected in an opportunistic manner, prioritizing those that provide high quality NMR data in the screens outlined above. Although some of the constructs that are generated may not be amenable to rapid structural analysis, it has been estimated that well over 50% of domains that are "trapped" by the process outlined above exhibit properties suitable for NMR or X-ray analysis. As these domains are derived from specific target genes associated with human diseases (discussed below) the chances of obtaining important new protein structures by this process are very high. Domains that provide diffraction quality crystals and which are not amenable to rapid analysis by NMR can be analyzed by X-ray crystallography.

**E. Computer Software And Related NMR Technologies For Fully Automated Analysis Of Protein Structures From NMR Data**

The present invention employs advanced NMR data collection and automated analysis technologies. These data collection and automated analysis technologies greatly accelerate the process of protein structure determination. Included within these technologies is a family of easy to use pulsed-field gradient triple resonance NMR experiments for rapid analysis of protein resonance assignments. See, for example, Montelione *et al., Proc. Natl. Acad. Sci. (U.S.A.) 86*:1519-1523 (1989); Montelione *et al., Biopolymers 32*:327-334 (1992); Montelione *et al., Biochemistry 31*:236-249 (1992); Lyons *et al., Biochemistry 32*:7839-7845 (1993); Rios *et al., J. Biomol. NMR 8*:345-350 (1996); Tashiro *et al., J. Mol. Biol. 272*:573-590 (1997); Shimotakahara *et al., Biochem. 36*:6915-6929 (1997); Laity *et al., Biochem. 36*: 12683-12699 (1997); Feng *et al., Biochem. 37*:10881-10896 (1998); and Swapana *et al., J. Biomol. NMR 9*:105-111.

These data collection and automated analysis technologies further include a fully automated strategy for determining NMR resonance assignments in proteins. Zimmerman *et al., Curr. Opin. Struct. Bio. 5*:664-673 (1995); and Zimmerman *et al., J. Mol. Biol. 269*:592-610 (1997).

Preferably, the data collection and automated analysis technologies of the present invention employ multiple-quantum coherences in triple resonance for enhanced sensitivity. Swapna *et al., J. Biomol. NMR 9*:105-111 (1997); Shang *et al., J. Amer. Chem. Soc. 119*:9274-9278 (1997).

**1. AUTOASSIGN: Artificial Intelligence Methods For Automated Analysis Of Protein Resonance Assignments**

Resonance assignments form the basis for analysis of protein structure and dynamics by NMR (Wüthrich, K., *NMR of Proteins and Nucleic Acids,* John Wiley & Sons, New York, New York (1986)), and their determination represents a primary bottleneck in protein solution structure analysis. However, the introduction of multi-dimensional triple-resonance NMR has dramatically improved the speed and reliability of the protein assignment process. Montelione *et al., J. Magn. Res. 83*:183-188 (1990); Ikura *et al., Biochem. Pharmacol. 40*:153-160 (1990); Ikura *et al., FEBS Letters 266*:155-158 (1990); Ikura *et al., Biochem. 29*:4659-4667 (1990), Tashiro *et al., J. Mol. Biol. 272*:573-590 (1997); Shimotakahara *et al., Biochem. 36*:6915-6929 (1997); Laity *et al., Biochem. 36*:12683-12699 (1997); Feng *et al., Biochem. 37*:10881-10896 (1998),

Preferably, the methods described herein employ AUTOASSIGN, an expert system that determines protein $^{15}$N, $^{13}$C, and $^{1}$H resonance assignments from a set of three-dimensional NMR spectra. Zimmerman *et al., Proceedings of the First International Conference of Intellegent Systems for Molcular Biology 1*:447-455 (1993); Zimmerman *et al., J. Biomol NMR 4*:241-256 (1994); Zimmerman *et al., Curr. Opin. Struct. Bio. 5*:664-673 (1995); Zimmerman *et al., J. Mol. Biol. 269*:592-610 (1997).

AUTOASSIGN has been copyrighted by Rutgers, the State University of New Jersey. Alternatively, the methods can employ one of the following expert systems for the automated determination of protein $^{15}$N, $^{13}$C, and $^{1}$H resonance assignments from a set of three-dimensional NMR spectra. These include a modified version of FELIX which is available from Molecular Simulation (San Diego, CA) (Friedrichs *et al., J. Biomol. NMR 4*:703-726 (1994),

CONTRAST which is available from the world wide web at <<www.bmrb.wisc.edu/macroo/soft_contrast.html>> (Olsen and Markley, *J. Biomol. NMR 4*:385-410 (1994), and a series of small programs described by Meadows, *J. Biomol. NWR 4*:79-86 (1994).

AUTOASSIGN is implemented in the Allegro Common Lisp Object System (CLOS) and requires a lisp compiler (available from Franz, Inc.) for execution. The software utilizes many of the analytical processes employed by NMR spectroscopists, including constraint-based reasoning and domain-specific knowledge-based methods. Fox *et al., The Sixth Canadian Proceedings in Artificial Intelligence* 1986); Nadel *et al.,* Technical Report, DCS-TR-170, Computer Science Department, Rutgers Univ. (1986); Kumar *et al., Artificial Intelligence Mag.,* Spring, 32-44 (1992).

Input to AUTOASSIGN includes a peak-picked 2D (H-N)-HSQC spectrum and the following seven peak-picked 3D spectra: HNCO, CANH, CA(CO)NH, CBCANH, CBCA(CO)NH, H(CA)NH, and H(CA)(CO)NH. This family of triple-resonance experiments can be used together with AUTOASSIGN to automatically determine extensive sequence-specific $^{1}$H, $^{15}$N, and $^{13}$C resonance assignments for several proteins ranging in size from 8 kD to 17 kD. Zimmerman *et al., J. Mol. Biol. 269*:592-610 (1997); Tashiro *et al., J. Mol. Biol. 272*:573-590 (1997); Shimotakahara *et al., Biochem. 36*:6915-6929 (1997); Laity *et al., Biochem. 36*:12683-12699 (1997); Feng *et al., Biochem. 37*:10881-10896 (1998). The program handles some of the very challenging problems encountered in automated analysis, including missing spin systems, spin systems that overlap even in the 3D spectra, and extra spin systems due to multiple conformations of the folded protein structure (e.g. X-Pro peptide bond cis/trans isomerization). Execution times on a Sun Sparc 10 workstation range from 16 to 360 sec, depending on the complexity of the problem analyzed by the program. Preferably, the NMR spectrometer is equipped with three channels and a fourth frequency synthesizer for carbonyl decoupling. Under another method the NMR spectrometer is equipped with four channels.

The AUTOASSIGN program provides for automated analysis of resonance assignments for atoms of the polypeptide backbone. Preferably, the AUTOASSIGN program provides for fully automated analysis of resonance assignments. Having established assignments for the backbone atoms of each amino acid in the protein sequence, it is relatively straightforward to extend from these to sidechain $^1$H and $^{13}$C resonance assignments using 3D HCCH COSY, HCCH-TOCSY, and HCC(CO)NH-TOCSY NMR experiments. Preferably, the AUTOASSIGN program handles automated analysis of these sidechain resonance assignments. It is additionally preferred that 3D $^{15}$N-edited NOESY and 3D $^{13}$C-edited NOESY data are collected and automatically analyzed to confirm the resonance assignments.

Under one method, AUTOASSIGN is designed to implement strategies that allow complete resonance assignments to be obtained with fewer NMR spectra. For example, sensitivity enhanced versions of HCCNH-TOCSY and HCC(CO)NH-TOCSY experiments can provide the complete set of information required for the determination of resonance assignments. This reduces the total data collection time required for determining backbone resonance assignments from the current 7 - 10 days to about half of this time. Zimmerman *et al.*, *J. Biomol. NMR 4*:241-256 (1994); Lyons *et al.*, *Biochemistry 32*:7839-7845 (1993).

Perdeuteration greatly lengthens the $^{13}$C transverse relaxation rates, allowing for higher sensitivity in these triple-resonance experiments. Grzesiek *et al.*, *J. Biomol. NMR 3*:487-493 (1993); Yamazaki *et al., Eur. J. Biochem. 219*: 707-712 (1994).

It has been demonstrated that significant sensitivity-enhancement (2 - 5 fold) can be obtained with triple-resonance experiments by perdeuteration of the protein samples. Preferably, the automated assignment strategy, described herein, will utilize $^2$H, $^{13}$C, $^{15}$N-enriched proteins prepared with protiated $^{15}$N-H amide groups, together with deuterium-decoupled triple resonance NMR experiments. Under one method, the amide NH group in the perdeuterated protein exchanges rapidly with the solvent $H_2O$ used in the course of the protein purification to yield the protiated $^{15}$N-H amide groups. This strategy can provide completely automated analysis of resonance assignments for the carbon and nitrogen skeleton of the protein. Having determined these assignments, analysis of resonance assignments for the attached hydrogen atoms can be completed using HCCH-COSY, HCCH-NOESY, and HCCH-TOCSY experiments. Correction factors for $^2$H-isotope shift effects for each carbon site of the 20 amino acids can be determined using data from model proteins. Preferably, the complete carbon resonance assignments in their protiated forms have already been determined for these model proteins.

Preferably, the methods utilize high temperature superconducting probes. First generation versions of these probes are currently being marketed by Varian NMR Inst. Inc. and Bruker Inst. Such probes in combination with the above-described technological advances reduce the time required for determining complete backbone and sidechain H, C, and N assignments to less than one week per domain.

## 2. Software For Automated Analysis Of Protein Structures From NMR Data

Having completed the resonance assignments for a particular protein, the next step of the structure determination process involves analyzing secondary structure (i.e. α-helices, β-sheets, turns, etc.). The chemical shifts themselves are often sufficient to allow identification of these features of secondary structure in the protein. Spera, *J. Amer. Chem. Soc. 113*:5490-5492 (1991); Wishart *et al., J. Biomol. NMR 6*:135-140 (1995). This information can be combined with other bioinformatics data derived from the protein sequence to narrow the number of possible mappings of the protein to known chain folds, and possibly even to identify the protein's biochemical function.

The principal sources of information used for the structure determination of protein domains are nuclear Overhauser effect (NOE) data arising from magnetic dipole-dipole interactions between hydrogen atoms in the structure of the protein. Interpretation of these data from multidimensional NOE spectroscopy (NOESY) spectra requires the resonance assignments, which will be obtained (as described above) in an automated manner. Preferably, the methods employ software for automated analysis of NOESY spectra and the generation of input files for rapid structure calculations using stimulated annealing of experimental constraint functions with molecular dynamics calculations.

The problems encountered in automatically analyzing NOESY spectra are due largely to spectral overlaps, i.e., it is often the case that several hydrogen atoms have very similar resonance frequencies. One of the preferred approaches to resolving this problem is to use 3D (or 4D) $^{15}$N- or $^{13}$C-resolved NOESY experiments (Clore *et al., Ann. Rev. Biophys. Biophys. Chem. 20*:29-63 (1991); Clore *et al., Prog. Biophys. Mol. Bio. 62*:153-184 (1994); Clore *et al., Methods Enzymol. 239*:349-363 (1994), in which one (or both) of the two protons involved in the NOE interaction is resolved in a third (or fourth) frequency dimension based on the frequency of the $^{15}$N or $^{13}$C nucleus to which it is covalently bound. Symmetry features of the 3D $^{13}$C-edited spectra can also be used to great advantage.

Another preferred approach to resolving ambiguities that arise in assigning NOESY cross peaks to specific pairs of interacting hydrogen atoms is to use the secondary structure (i.e. α helix, β strand, etc.) to predict NOEs that are expected and to use these structural predictions to guide the analysis of NOESY spectra. Meadows *et al., J. Biomol. NMR 4*:79-96 (1994).

A third preferred approach is to use a low-resolution structure of the protein obtained in a first pass analysis of the uniquely assigned NOESY cross peaks to identify candidate assignments of the remaining unassigned NOESY cross peaks which are inconsistent with the low-resolution structure.

The approaches outlined above are those that are routinely used by a human expert in the analysis of NOESY spectra. Under the preferred method, the reasoning processes of those approaches are encoded into the software.

Preferably, the software program is a $C^{++}$ program. AUTO_STRUCTURE is a $C^{++}$ program that analyzes 2D and 3D NOESY spectra to identify unique NOESY crosspeak assignments (Gaetano Montelione, Y. Huang and Robert Tejero (Rutgers, The State University of New Jersey)). The program then uses these crosspeak assignments to create distance-constraint input files for simulated annealing structure calculations. AUTO_STRUCTURE can also use a low-resolution (or homology-modeled) structure of the protein to filter the list of NOESY crosspeaks that are not uniquely assigned, removing potential-NOE assignments that are severely inconsistent with the low-resolution structure. AUTO_STRUCTURE propagates the structural constraints imposed by the uniquely assigned NOEs to determine assignments of otherwise ambiguous NOEs. AUTO_STRUCTURE can successfully analyze NOESY spectra and, in an iterative fashion, automatically generate 3D structures of simple polypeptides. Other auto structure programs for NOESY analysis that can be used in the present invention include GARANT (Wuthrich (ETH, Zurcih, Germany), incorporated by reference in its entirety), ARIA (Michael Nilges, *J. Mol. Biol. 245:645*-660 (1995), incorporated by reference in its entirety) and NOAH (Mumenthaler and Braun, *J. Mol. Bio. 254*:465-420 (1995),

Preferably, the auto structure program provides for automated analysis of protein or protein domain structures. Under a more preferred method, the auto structure program further contains sophisticated reasoning processes which can assist in resolving ambiguous NOESY crosspeak assignments in the absence of even a low resolution 3D structure. Preferably, this includes (i) the propagation of structural constraint information inherent in the secondary structure analysis stemming from the resonance assignments and (ii) the application of pattern recognition algorithms.

## F. Mapping New Domain Structures To Proteins In The Protein Data Base (PDB) With Similar Structures And Biochemical Functions

Preferably, the resulting domain structures derived from NMR or X-ray crystallographic analyses are compared with the PDB or other suitable databases of known protein structures using an algorithm for 3D-structure homology matching. Examples of publicly available PDBs suitable for use include the Protein Data Base (PDB), which can be found at http://www.pdb.bnl.gov/. Algorithms for 3D-structure homology matching suitable for use include the DALI analysis program (Holm *et al., J. Mol. Biol. 233*:123-138 (1993), herein incorporated by reference), the CATH analysis program (Orengo, C. A., *Structure 5*:1093-1108 (1997), VAST (http://www.ncbi.nlm.nih.gov/Structure/vast.html; Gibrat *et al., Current Opinion in Structural Biology 6*: 377-385 (1996); and Madej *et al., Proteins 23*: 356-369 (1995), or similar algorithms for 3D-structure homology matching.

DALI compares "contact maps" of protein structures to identify homologies in 3D structure and provides a list of PDB entries with high match scores. Based on current "hit" rates by newly-determined structures against already known folds (Holm *et al., Methods Enzymol. 266*:653-662 (1996); Holm *et al., Science 273*:595-603 (1996), it is expect that greater than 50% of the structures will show significant structural and functional homology to proteins of known structure and function.

In order to facilitate and enhance the ability to identify common biochemical functions for these DALI hits, it is preferable to develop a structure-function knowledge base (Figure 1), correlating each protein structure in the PDB with the set of biochemical functions that have been associated with that protein in the published scientific literature. Where information is available, this knowledge base will also correlate the portions of these known protein structures with corresponding specific biochemical functions (e.g., enzymatic active sites or nucleic-acid binding loops). This fold-function knowledge base is applicable to a wide range of structural bioinformatics applications, and of significant utility to the nascent industry of structural bioinformatics.

Once novel protein domains with clear homologies to better-characterized counterparts have been identified, the proposed functions can be validated using biochemical assays. For example, if a protein looks like a member of the galactosyl transferase family, the protein will be tested for radioactive UDP-galactose (or other carbohydrate) binding, if it looks like a lipase, the protein will be tested for lipid binding and/or hydrolysis activity, and so on.

## G. Integration Into A Large-Scale, High-Throughput "Engine" For Structural And Functional Analysis Of Hundreds Of Human Genes

One preferred method, provides for a "structure - function analysis engine" capable of high-throughput discovery of biochemical functions of new human disease genes and genes of unknown function.

Using conventional methodology, the skilled artisan may be able to determine the 3D structure of one protein per year. However, using the methodology described herein, it is possible to determine the 3D structure of far greater than one protein per year. Under optimal conditions, the methods will enable a properly equipped laboratory to generate the 3D structure of one protein per month per NMR machine. As used herein, "high-throughput" refers to the ability to determine the 3D structures of protein domains of unknown function at a rate which is faster than the rate at which a skilled artisan could determine a protein structure using traditional methodologies.

One of the central features of the present invention is that it is highly scaleable. Under one of the preferred embodiments, the high-throughput "engine" consists of a dedicated laboratory staffed with artisans skilled in relevant arts (e.g., NMR and X-Ray crystallography, molecular biology, biochemistry, etc.). Preferably, such a laboratory is further equipped with state of the art equipment for the sequencing, sub-cloning, expression, purification, screening and analysis of the protein domains of interest. The rate limiting component of this high-throughput "engine" is the number of NMR machines within the laboratory. Thus, the rate at which protein domains can be characterized will increase with the addition of additional NMR machines. Unlike conventional methodology, the present invention provides a method for determining the 3D structure of unknown protein domains whose rate is not solely dependent on the number of artisans skilled in 3D protein structure determination.

The rate of domain characterization increases as each of the tasks which are presently conducted by hand are automated. For example, under one method, the parsing of the unknown gene into its component domains is facilitated through the use of advanced sequence analysis algorithms. Under another method, the rate of domain characterization is increased through the use of improved computer software for the automated analysis of NMR datapoints.

Although the present invention is drawn to using NMR to determine protein structure and function, it is to be understood that a person of skill in the art could perform similar analysis using X-ray crystallography to practice the present invention. Shapiro and Lima, *J. Structure 6*:265-267 (1998); Gaasterland, *Nature Biotech. 16:*625-627 (1998); Terwilliger *et al. Prot. Sci. 7*:1851-1856 (1998); Kim, *Nature Structure Biology (Synchrotron Supp.):* 643-645 (1998),

### III. SPECIFIC GENE TARGETS

Preferably, the specific gene targets that will be analyzed will be genes that are known to be involved in human diseases but for which the biochemical function and three-dimensional structures of the proteins encoded by the genes are not available. These protein domains will be analyzed using the high-throughput "structure - function analysis engine". The resulting structural and functional information will be critical in developing pharmaceuticals targeted to these human gene products.

Although the present invention is principally drawn to human genomic, cDNA and mRNA sequences, it is to be understood that the present invention is generically applicable to genomic, cDNA and mRNA sequences of any living organism or virus.

Although the methods are capable of determining the function of any given protein or protein domain, the preferred biomedical gene targets include Alzheimer's β peptide precursor protein (APP). Additional preferred biomedical gene targets include but are not limited to those genes implicated in neoplastic, neurodegenerative, metabolic, cardiovascular, psychiatric and inflammatory disorders. The genomes/genes of infectious agents, such as pathogenic microbes, pathogenic fungi and pathogenic viruses, are also preferred targets for study.

By focusing on medically important diseases, it is anticipated that the present invention will greatly facilitate the identification of protein targets for subsequent drug discovery efforts.

Having now generally described the invention, and methods described herein, the same will be more readily understood through reference to the following examples which are provided by way of illustration and are not intended to be limiting on the present invention.

### EXAMPLE 1

### PARSING OF THE APP GENE INTO DOMAIN-ENCODING REGIONS

### A. Parsing By The Exon Phase Rule

The human amyloid beta peptide precursor (APP) protein gene (Yoshikai *et al.*, *Gene 87*:257-263(1990)) was subjected to a parsing analysis with respect to the phases of its exon-exon boundaries:

| Exon-exon boundary | Phase |
| --- | --- |
| 1 - 2 | 0 |
| 2 - 3 | 0 |
| 3 - 4 | 1 |
| 4 - 5 | 0 |
| 5 - 6 | 2 |
| 6 - 7 | 1 |

(continued)

| Exon-exon boundary | Phase |
|---|---|
| 7 - 8 | 1 |
| 8 - 9 | 1 |
| 9 - 10 | 0 |
| 10 - 11 | 0 |
| 11 - 12 | 0 |
| 12 - 13 | 0 |
| 13 - 14 | 1 |
| 14 - 15 | 1 |
| 15 - 16 | 1 |
| 16 - 17 | 0 |
| 17 - 18 | 0 |

Using the exon phase rule, only exons or exon combinations that start or stop in the same phase are allowed. For example, exon 7 or exons 7+8 are potential domain encoding regions with phase 1 boundaries. Likewise, exon 10, exons 10+11, and exons 10+11+12 would be potential domain encoding regions with phase 0 boundaries.

**B. Exon Phase And The Alternative Splicing Rule**

The APP gene is reported to be alternatively spliced. The longest polypeptide encoded by the APP gene is 770 amino acids long, and shorter isoforms exist that are missing the amino acids encoded by exons 7.8, and/or 15 (Sandbrink *et al., Ann. NY Acad Sci. 777*:281-287 (1996)). All of these exons which are alternatively spliced are bounded by phase 1 termini. Alternative splicing must be done in such a way as to not disrupt the integrity of the holoprotein (i.e., without destroying essential folding information). The fact that all alternatively spliced exons have phase 1 termini implies that domain boundaries may be congruent with phase 1 exon boundaries, that is, phase 1 exon boundaries in this particular gene are candidate boundaries of domain encoding regions.

**C. Setting The Phase With Known Internal Domain Structures**

Exon 7 of APP is known to encode a complete domain for a Kunitz-type serine protease inhibitor (Hynes *et al., Biochemistry 29*:10018-10022 (1990)). The Kunitz inhibitor is a domain that has been combinatorially shuffled around in various genes during evolution (Patty, L. *Curr. Opin. Struct. Biol. 1*:351-361 (1991)), and for the reasons given above it would have to be inserted only into proteins with other domains of the same phase in order to not disrupt gene expression. Therefore, this analysis is also consistent with APP being composed of domains which are bounded by phase 1 exon termini.

**D. The "N-Terminus First" Strategy Of Parsing**

In order to reduce the combinatorial complexity of the parsing problems, an "N-terminus first" strategy is preferred. In this parsing strategy, expression constructs of putative domains arc made starting from the N-terminus of the protein and extending to the likely C-termini as predicted by the above rules. These constructs are put through the "domain trapping" test of the present invention in order to identify the first N-terminal domain. Then, once the first N-terminal domain is identified, a second set of constructs commencing from the C-terminus of the first N-terminal domain is made, and so on.

In the case of APP, the N-terminus of the protein starts with exon 2 because exon 1 encodes a signal peptide. Therefore, the possible domain constructs that ended in phase 1 boundaries were exons 2-3 and exons 2-6 (exon 7 was known to encode the Kunitz inhibitor domain). By the domain trapping criteria exons 2-3 were found to encode the first N-terminal domain, so a second construct composed of exons 4-6 was made and found to contain the second domain of APP, and so on. A summary of the APP domains identified by this combination of parsing and domain trapping is given below:

| Domain | Encoding Exons |
|---|---|
| 1 (N-terminal domain) | 2-3 |
| 2 | 4-6 |

(continued)

| Domain | Encoding Exons |
|---|---|
| 3 (Kunitiz inhibitor) | 7 |
| 4 | 8 |
| etc. | |

## EXAMPLE 2

## EXPRESSION AND PURIFICATION OF AN ISOLATED DOMAIN

The putative domain regions identified in Example 1 are sub-cloned into the secretion-based protein A fusion expression system and purified. Nilsson *et al.*, *Methods Enzymol.185*:144-161 (1990).

## EXAMPLE 3

## EXPRESSION AND PURIFICATION OF AN ISOLATED DOMAIN FOR NMR ANALYSIS

### A. Protein Expression

E. coli strain RV308 is used as the bacterial expression host. Competent RV308 cells are transformed with pHAZY plasmid containing the NTD 2-3, Z domain insert. Cells are grown overnight at 37°C on LB agar plates supplemented with 100 g/ml ampicillin (Sigma). Fresh transformants arc used to inoculate seed cultures in 2 x TY media (16 g/l typtone, 10 g/l yeast extract, and 5/g NaCl) supplemented with 100 $\mu$g/ml ampicillin. Cultures are grown overnight at 30°C in 250 ml baffled flasks. A ratio of 1 to 25 is used to inoculate expression cultures. For 1 liter of MJ media expression culture (2.5 g/l $^{15}NH_4$ sulfate (>98% purity), 0.5 g/l sodium citrate, 100 mM potassium phosphate buffer, pH 6.6, supplemented with 5 g/l $^{13}$C-glucose (>98% purity), 1 g/l magnesium sulfate, 70mg/l thiamine, 1 ml of 1000 x trace elements solution, 1 ml of 1000 x vitamin solution, and 100 mg/l ampicillin), 40 ml of seed culture is spun down by centrifugation. Bacterial pellets are washed, resuspended in fresh MJ media, and used to inoculate expression cultures. Cultures are grown at 30° in 2 l baffled flasks and induced at OD$^{55}$ 0.9 - 1.0 with indole acrylic acid to a final concentration of 20 mg/l. Cultures are harvested 15 hours after induction by centrifugation. Bacterial pellets are stored at 20°C until purification.

### B. Protein Purification

Bacterial cells are resuspended in 100 m of 25 mM Tris, pH 8.0, 5 mM EDTA. 0.5% Triton X-100 and sonicated continuously for 9 minutes. Released inclusion bodies are pelleted by centrifugation and washed with fresh sonication buffer. Inclusion bodies were then solubilized with 7 M guanidine HCl and 10 mM DTT. Centrifugation is used to pellet any undissolved material. Guanidine and DTT are then diluted twenty fold by dialysis against twenty volumes of 10 mM HCl.

IgG affinity purification is used to purify the NTD 2-3, Z domain fusion from any contaminating proteins. The 10 mM HCl protein solution is neutralized to > pH 7 with 1 M Tris, pH 8.0. The sample is then applied to an IgG sepharose column (Pharmacia) pre-equilibrated with TST buffer. The column is washed with 10 bed volumes of TST (50 mM Tris, 150 mM NaCl, and 0.05% TWEEN™ 20) followed by 2 bed volumes of 5 mM ammonium acetate, pH 5.0. Finally, the protein is eluted with 0.5 M acetic acid, pH 3.4. In preparation for refolding, the protein eluate is neutralized to pH 8.0 with solid Tris, and an equal volume of 7 M guanidine is added to bring the final guanidine concentration to 3.5 M.

Refolding of the protein is carried out by using dialysis to slowly dilute out the guanidine HCl while slowly introducing the refolding buffer. Firstly, Spectra/POR dialysis tubing with a MWCO of 6000-8000 is soaked overnight in water in order to remove glycerol. Next, the protein solution is loaded into the primed tubing and dialyzed against fresh refolding buffer. The dialysis reaction is incubated for two days at 4°C with magnetic stirring. Refolded protein is then concentrated using an IgG sepharose column pre-equilibrated with TST buffer. Bound protein is eluted with 0.5 M acetic acid and collected in fractions in order to keep the volume as low as possible. Refolded fusion protein is then further purified by gel filtration on a Pharmacia Superdex 75 FPLC column using 300 mM ammonium bicarbonate, 0.1 mM copper sulfate as the buffer. Fractions corresponding to the fusion protein are pooled, and the protein is quantitated using the optical density at 280 nm.

Cleavage of the fusion protein is carried out using Genenase I (NEB), a variant of subtilisin BPN'. Fusion protein is buffer exchanged into Genenase buffer, 20 mM Tris, pH 8.0. 200 mM NaCl, 0.02% NaN$_3$, using an Amicon stir cell. The protein concentration is adjusted to 2 mg/ml and Genenase is added to a concentration of 0.2 mg/ml. The reaction is incubated at room temperature for 4 days and the extent of cleavage was followed using SDS-PAGE. Cleaved NTD 2-3 is separated from uncleaved fusion and Z domain by passing the solution over an IgG column and collecting the unbound NTD 2-3 in the flow through. The NTD is then purified from Genenase by gel filtration on a Pharmacia Superdex 75 FPLC column using 300 mM ammonium bicarbonate. 0.1 mM copper sulfate as the buffer.

## EXAMPLE 4

### DOMAIN TRAPPING:CHARACTERIZATION OF AN ISOLATED DOMAIN

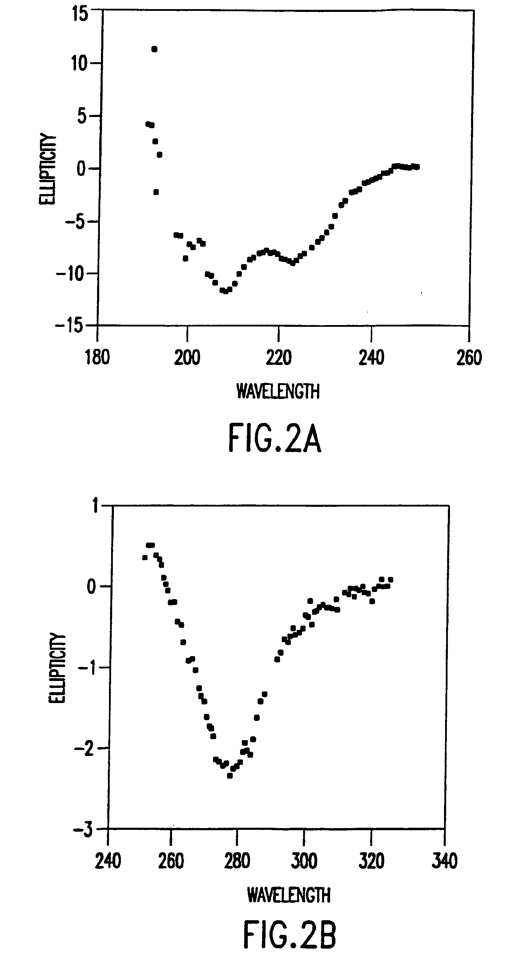

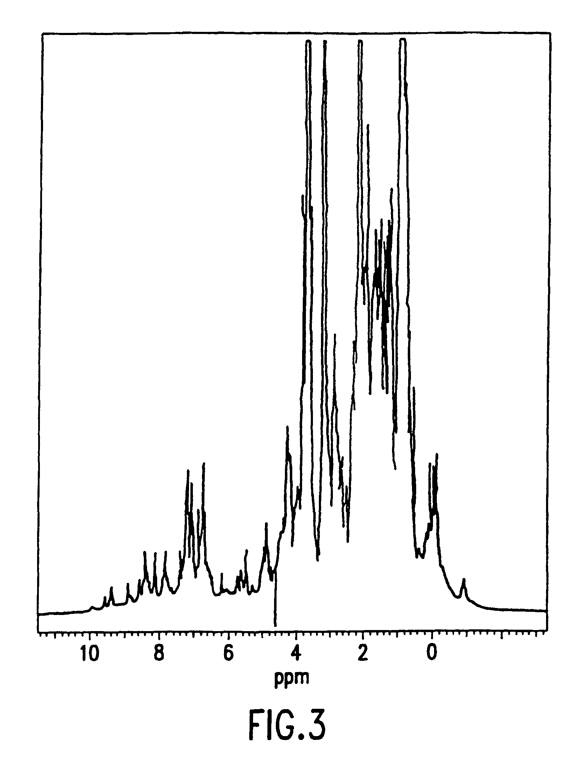

Characterization of an isolated domain (NTD2-3) from the Alzheimer's amyloid precursor protein (APP) by circular dichroism measurements in the far UV shows an ellipticity minimum at 222 nm, indicative of $\alpha$-helical secondary structure (Figure 2A). Of even greater significance. CD measurements at longer wavelengths reveal a clear signal in the aromatic region around 280 nm, consistent with the presence of Trp, Tyr, and Phe chromophores in an ordered environment such as would be expected in the hydrophobic core of a folded protein (Figure 2B). A moderately concentrated solution (~100 $\mu$M) of the isolated N-terminal domain is further characterized by one-dimensional $^1$H-NMR. The isolated recombinant APP N-terminal domain exhibits high dispersion of the proton resonances, which is a signature of well-folded polypeptides (Figure 3).

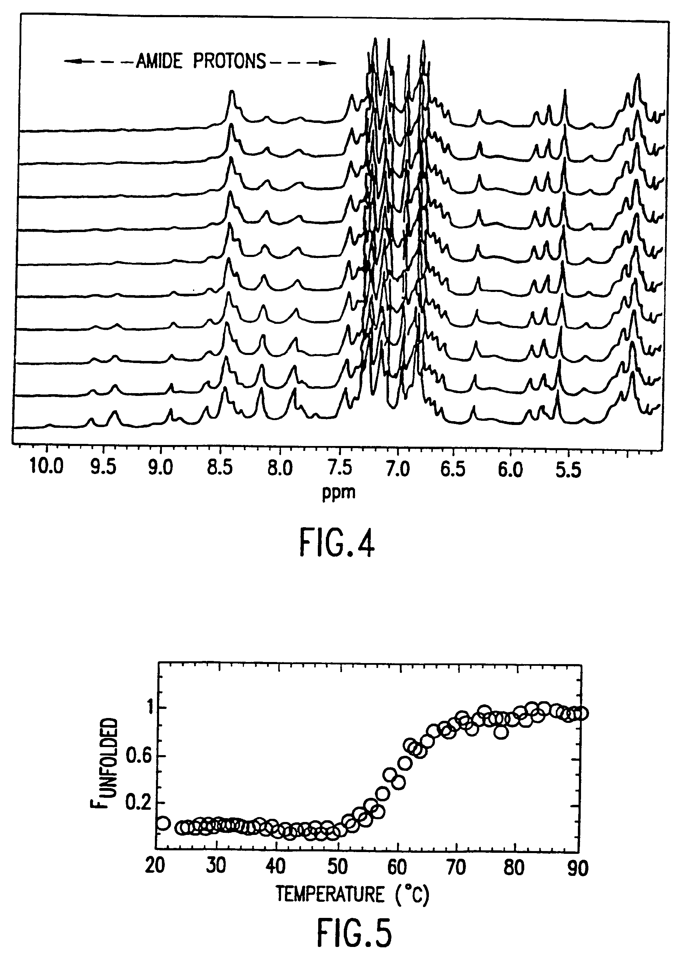

A time-course of amide hydrogen-deuterium exchange measurements is performed. From this, it is observed that many backbone NH groups exhibit significant protection, indicating hydrogen-bonded secondary structure stabilized by tertiary interactions consistent with a well-folded domain structure (Figure 4). Finally, thermal denaturation experiments, monitored by intrinsic tryptophan fluorescence, are performed. These experiments show that the recombinant APP NTD2-3 domain undergoes a cooperative thermal unfolding transition, with a $T_m$ of approximately 60° C, indicative of a compact domain structure (Figure 5).

Thus, using biophysical data alone, it is demonstrated that the NTD2-3 domain of APP, encoded by exons 2 and 3, is expressed as a well ordered tertiary structure. Chiang *et al., Neurobiol. Aging,* Supplement Vol. 17, No. 4S, abstract 393 (1996). Similar studies indicate that the next APP N-terminal domain is encoded by exons 4-6. the third (Kunitz) domain by exon 7, and so on.

## EXAMPLE 5

### NMR CHARACTERIZATION OF THE NTD 2-3 DOMAIN

For NMR studies NTD 2-3 is concentrated to concentrations greater than 10 mg/ml. Gel filtration pure NTD 2-3 is first buffer exchanged into a NMR compatible buffer, 20 mM potassium phosphate, pH 6.5 using an Amicon stir cell. The protein solution is then concentrated to an appropriate volume based on the amount of protein present using the Amicon 50 and Amicon 3 stir cells. The final protein concentration is confirmed by optical density at 280 nm.

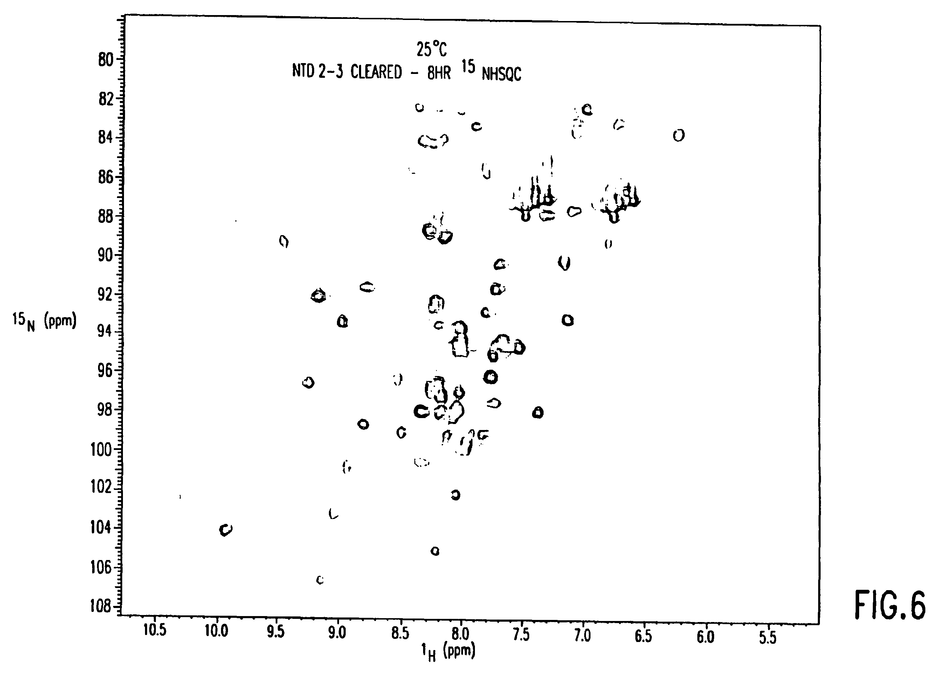

NMR $^{15}$N-HSQC spectra is collected on a Varian Unity 500 spectrometer. The $^{15}$N-HSQC spectral analysis is shown in Figure 6. The good dispersion in both the $^{15}$N and $^1$H dimensions demonstrate that this is a folded domain that has been "trapped" by the presently described methods.

## EXAMPLE 6

### COMPARISON OF THE NMR STRUCTURE OF CSPA WITH OTHER PROTEINS

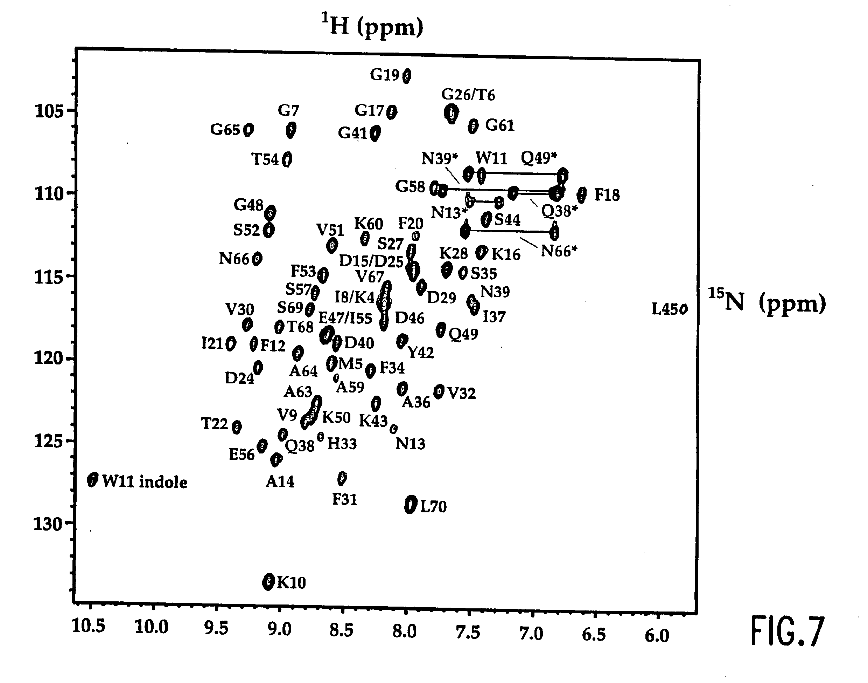

Recombinant CspA is expressed and purified using the protocol essentially as described by Chatterjee *et al., J. Biochem. 114*:663-669 (1993), and Feng *et al., Biochemistry 37*:10881-10896 (1998). The purified CspA protein is prepared for NMR analysis by dialysis against a buffer containing 50 mM potassium phosphate and 1 mM NaN$_3$, pH 6.0 and the sample is analyzed using a Varian Unity 500 spectrometer equipped with three channels and a fourth frequency synthesizer for carbonyl decoupling as described by Feng *et al., Biochemistry 37*:10881-10896 (1998). Figure 7 provides the 2D $^{15}$N-$^1$H$^N$ HSQC spectrum of CSPA at pH 6.0 and 30°C.

The collected spin resonances are analyzed using AUTOASSIGN. The input for AUTOASSIGN includes peaks from 2D $^{15}$N-$^1$H$^N$ HSQC and 3D HNCO spectra along with peak lists from three intraresidue (CANH, CBCANH and HCANH) and three interresidue (CA(CO)NH, CBCA(CO)NH and HCA(CO)NH) experiments, which correlate with the C$^\alpha$. C° and H$^\alpha$ resonances of residues *i* and *i*-1 respectively. The results of the AUTOASSIGN analysis of the peak picked 2D and 3D NMR spectra are summarized in Table 1.

Side chain resonance assignments are obtained using PFG HCCNH-TOCSY and PFG HCC(CO)NH-TOCSY and homonuclear TOCSY experiments recorded with multiple mixing times of 22, 36, 45, 54, 71 and 90 ms according to the method of Celda and Montelione. *J. Magn. Reson. B101*:189-193 (1993), incorporated by reference in its entirety.

Interatomic distance constraints are derived from three NOESY data sets 2D NOESY and 3D $^{15}$N-edited NOESY-HSQC spectra recorded with a mixing time of $t_m$ of 60 ms of a CspA sample dissolved in 90% $H_2O$/10% $^2H_2O$ and a 2D NOESY spectrum is recorded with a mixing time $t_m$ of 50 ms of a sample dissolved in 100% $^2H_2O$. The intensity of the NOESY-HSQC spectrum is corrected for $^{15}$N relaxation effects, and the cross-peak intensities are converted into interproton distance constraints.

| Table 1 | | | | | |
|---|---|---|---|---|---|
| Summary of AUTOSSIGN Analysis for CspA Triple-Resonance NMR Data | | | | | |
| Residues | 69 | | Number of assignments (expected) | AUTOASSIGN analysis | Manual analysis |
| | | | Backbone | | |
| GSs expected | 66 | | $H^N$ | 65 | 66 |
| GSs observed | 67 | | $H^\alpha$ | 77 | 79 |
| Degenerate GS roots | 8 | | $^{15}$N | 65 | 66 |
| Assigned GSs | 65 | | $^{13}C^\alpha$ | 67 | 69 |
| Extra GSs | 2 | | $^{13}C^\alpha$ | 64 | 66 |
| Assigned residues | 68 | | $^{13}C^\beta$ | 49 | 59 |
| Percent assigned residues | 99% | | Side chain | | |
| Execution times (sec.) | 10 | | $^{15}$N | 6 | 6 |
| | | | $H^N$ | 11 | 11 |

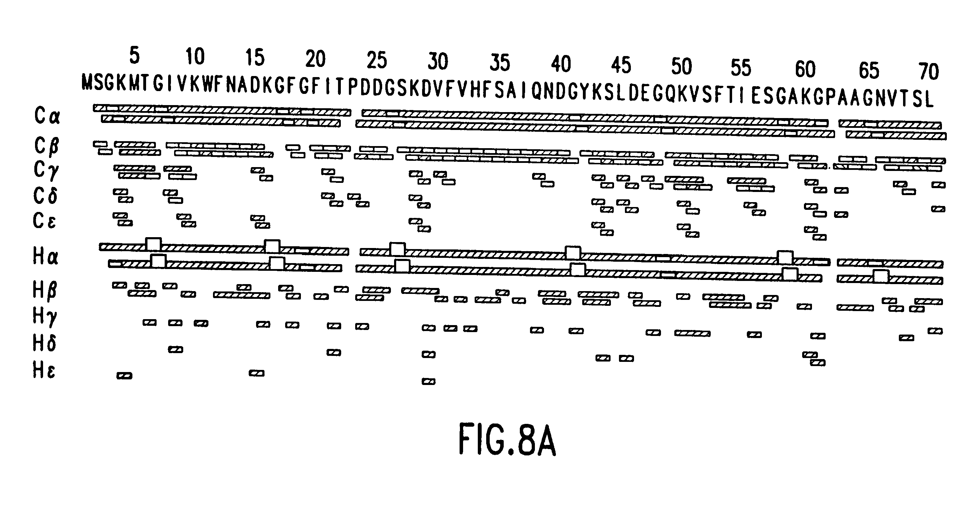

Stereospecific assignments of methylene $H^\beta$s are made by analysis of local NOE and vicinal coupling constant data using the HYPER program. HYPER is a conformational grid search program used for determining stereospecific $C^\beta H_2$ methylene proton assignments and for defining the ranges of dihedral angles $\phi$, $\psi$, $\chi'$ that are consistent with the local experimental NMR data for each amino acid in a polypeptide (Tejero *et al., J. Biomol. NMR* (in press). The secondary structural elements of CspA are summarized in Figure 8. From this information, five β-strands corresponding to polypeptide segments of residue 5-13, 18-22, 30-33, 50-56 and 63-70 are identified.

The average number of distance constraints per residue is 10.4. Dihedral angel constraints are obtained from the HYPER program. Structure generation calculations are carried out with DIANA, version 2.8 TRIPOS, Inc.) using R8000 processor in a Silicon Graphics Onyx workstation (Braun and Go. *J. Mol. Biol. 186*:611-626 (1985), and Guntert *et al., J. Mol. Biol. 169*:949-961 (1983).

From this NMR data set, the solution structure of CspA is reasonably well defined. Using the refined CspA coordinates defined by the present invention, structural database searches of the Protein Data Base (PDB) are performed with the DALI program. This search is able to identify a list of proteins or domains of structural homologues. Identified structural homologues of CspA exhibiting similar biochemical function include the RNA binding domain of *E. coli* polyribonucleotide nucleotidyltransferase, the human mitochondrial ssDNA-binding protein. *E. coli* translation initiation factor 1, the ssDNA-binding protein from gene V of filamentous bacteriophages M13 and f1, the ssDNA-binding protein from *Pseudomonas* phage Pf3, elongation factor G from *Thermus thermophilus*, a domain of *E. coli* lysyl tRNA synthetase, a domain of yeast tRNA synthetase, human replication protein A, staphylococcus nuclease, and a domain of *E. coli* topoisomerase I. Although the function of CspA was already know, the present Example has illustrated the use of the present invention.

As the present invention describes, a person of skill in the art is able to take a polypeptide of unknown function, express and purify a stable peptide domain encoded by the polypeptide, determine the NMR 3D structure of that expressed domain and predict the function of that domain by comparing the structure of that domain against known structures having known functions. This represents a fundamental paradigm shift in the study of proteins.

## EXAMPLE 7

## AUTOMATED ANALYSIS OF PROTEIN STRUCTURES FROM NMR DATA

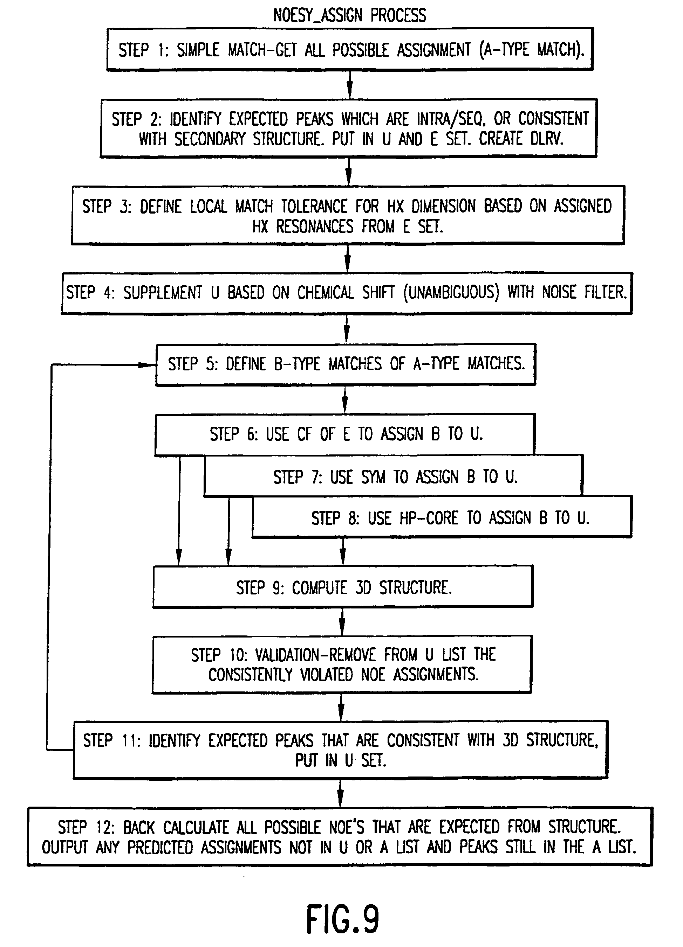

Figure 9 outlines the constraint reasoning system of the present invention which automatically generates protein structures from NMR data. Briefly, the constraint reasoning system is based on automated analysis of secondary structure, prediction of hydrophobic core contacts, and iterative analysis of contact frequencies. The constraint reasoning generates reliable initial chain folds even when the chemical shift information alone provides few unambiguous NOESY cross peak assignments.

In the first step, a Simple Match is performed to determine all possible assignments (A-type matches) for each spectra. In the second step, the expected peaks which are consistent with secondary structure, or which are intra/seq are identified. These peaks are placed in an experimental (E) and an unknown (U) set. The expected peaks are further used to create a dynamically locally referenced values (DLRV) for H and HX (local referencing). The DRLV for each atom in each dimension includes the original chemical shift value plus any additional chemical shift values derived from the E set. If only one expected match is found for a given peak, that peak is put into U and E set. If more than one expected match is found for a given peak (B-type expected matches), those expected matches are also put into U and E set.

In the third step, the local match tolerance for HX dimension is defined. The local match tolerance for HX dimension is based on assigned HX resonance from E set. HX resonance is performed as described by Koide *et al., J. Biomol. NMR 6*:306-312 (1995); Bai *et al., Proteins 20*:4-14 (1994): and Englander and Mander, *Annu. Rev. Biophys. Biomol. Struct. 21*:243-265 (1992).

In the fourth step. U peaks are supplemented based on chemical shift (unambiguous) data filtered through a noise filter. The noise filter reduces the background noise by eliminating peaks having an intensity of <0.05% of the highest intensity of the real intra peaks. Thus, a tighter match tolerance to chemical shift list is created by the noise filter makes than the list created by the Simple Match of step 1.

B-type matches, a subset of A-type matches for each spectra, are defined in step 5. The B-type matches for a given peak are defined by ordering the A-type matches based on the size of the match value. The match value is computed as follows:

$$MV = \min(\Delta HX + \Delta X/10 + \Delta H)$$

where $\Delta H = H_{obs} - H_{DCSL}$; $\Delta HX = HX_{obs} - HX_{DCSL}$; $\Delta X = X_{obs} - X_{DCSL}$; and $H_{DCSL}$, $HX_{DCSL}$ and $X_{DCSL}$ are sets of dynamically locally referenced values (DLRV) for the H, HX, and X dimensions, respectively. All possible matches with $\gamma \leq 0.01$ are chosen, where $\gamma = | MV - (\Delta HX + \Delta X/10 + \Delta H) |$.

In step 6, the Contact Frequency (CF) of E is used to assign B-type matches to U set. A contact bin is created from all E's. If a peak in B is in the contact bin, it is assigned to U. Otherwise, it is assigned to T-type matches. In step 7, SYM, a constraint satisfaction program, is used to assign B-type matches to U set. If a peak in B has symmetry to another peak in B. both are assigned to U set as T-type assignments. SYM modeling is performed utilizing the method described by Gdaniec *et al., Biochemistry 37*:1505-1512 (1998); Easterwood and Harvey, *RNA 3*:577-585 (1997); Laine and Hall, *Biochemistry 35*:13586-13596 (1996): Ericson *et al., J. Mol. Biol. 250*:407-419 (1995): and Foucrault and Major. *ISMB 3*:121-126 (1995). In step 8. HP-CORE. which predicts buried residues, is used to assign B-type matches to U set. A HP-CORE contact bin is created from all B's. If the contract frequency (CF) of the HP-CORE contact bin is > N, all peaks in this bin are assigned to U as T-type assignments. N is a heuristic value that is scale with the number of NOESY spectra available.

The 3D structure of the protein is computed in step 9. First, the structure calculation program is calibrated, where the distance of D-type peaks are derived from their intensity and the distance of T-type peaks are = 5.0Å. The structure calculation program is then run. The 10 best results, from a family of 50 3D structures are selected. For each of the 10 best results, the $S(\phi)$, $S(\varphi)$, $\sigma(i,j)$ matrix, bb root mean square deviation (RMSD) are calculated where records with a $S(\phi) < 0.7$ and $S(\varphi) < 0.7$ are excluded. If the rmsd is too large, further analysis is stopped. If the rmsd is < 1Å, the analysis continues with step 12. If it has progress, analysis continues with step 10. If there isn't any more progress, analysis proceeds with the next cycle (decrease O). Disordered regions - order (i.j) are identified from O. If ($<S - O> \geq 0$ and $(\sigma(i,j) - 2/O) \leq 0$) and order(i,j) = 1, then the region is ordered. If order(i,j) = 0, then the region is disordered.

In the validation step, step 10, peaks that consistently violated NOE assignments are removed from U list. If the peak is greater than the Violation Parameter (V), it is assumed that the assignment is wrong. If order(i,j) = 1, then V = I and if order(i,j) = 0. then V = 2. If the $v_{mm}(i,j)$ is greater then V and it is a T-type assignment, it is deleted from the assignment list. If it is a D-type assignment, it is downgraded to a T-type assignment and assigned an alternate assignment of <d> <5Å. If a peak has more than one T-type assignment and only one of the peaks has violated V, it is reassigned as a D-type assignment.

In step 11. expected peaks that are consistent with 3D structure are identified and placed in U set. It is assumed that if the peak is in an ordered region and it is greater than the Distance Cutoff (D), it is an incorrect assignment. If (order(i,j) = 1), then D = 5 + rmsd*2 and Dmin = 5.5Å. N, the number of possible assignments left, is put into U set. If rmsd > 2, then $N \leq 2$. If rmsd > 1, then $N \leq 3$. For any other rmsd value, $N \leq 4$. Any assignment with a $d_{mm}(i,j) > D$ in ordered region is removed from A list. If N possible assignments arc left, they are put into U set as T-type assignments.

In set 12, all possible NOE's that are expected from the structure are back calculated. Any predicted assignments not in U or A list and any peak still in A list are outputted. For each cycle, a Contact Map (assignment, structure), Connectivity Map, Structures, Assignments (ordered by intra, seq, mid, long range), S($\phi$), S($\varphi$), σ(i,j) matrix, and bb rmsd are outputted.

### EXAMPLE 8

### AUTOMATED GENERATION OF 3D STRUCTURES

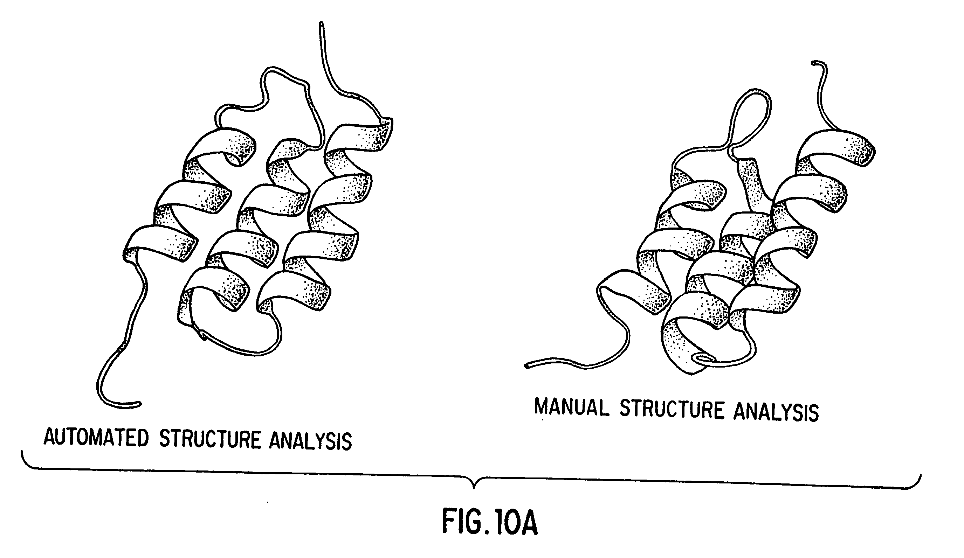

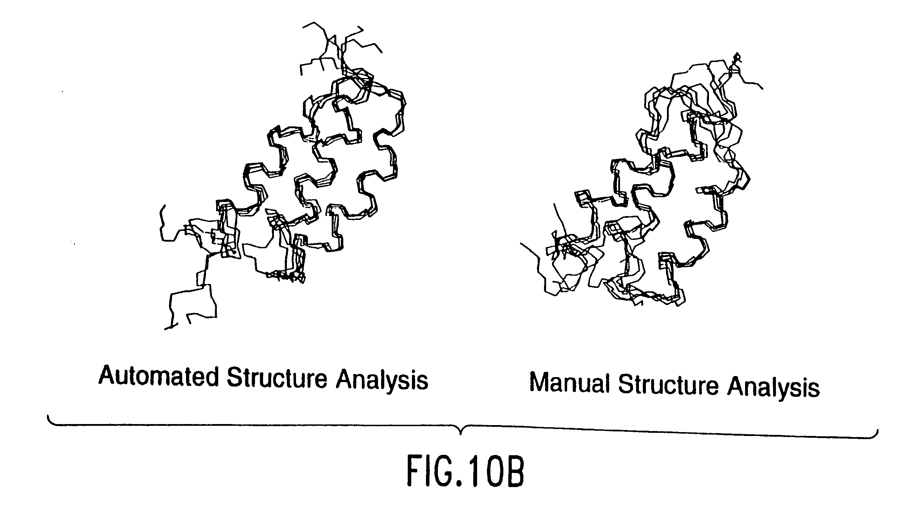

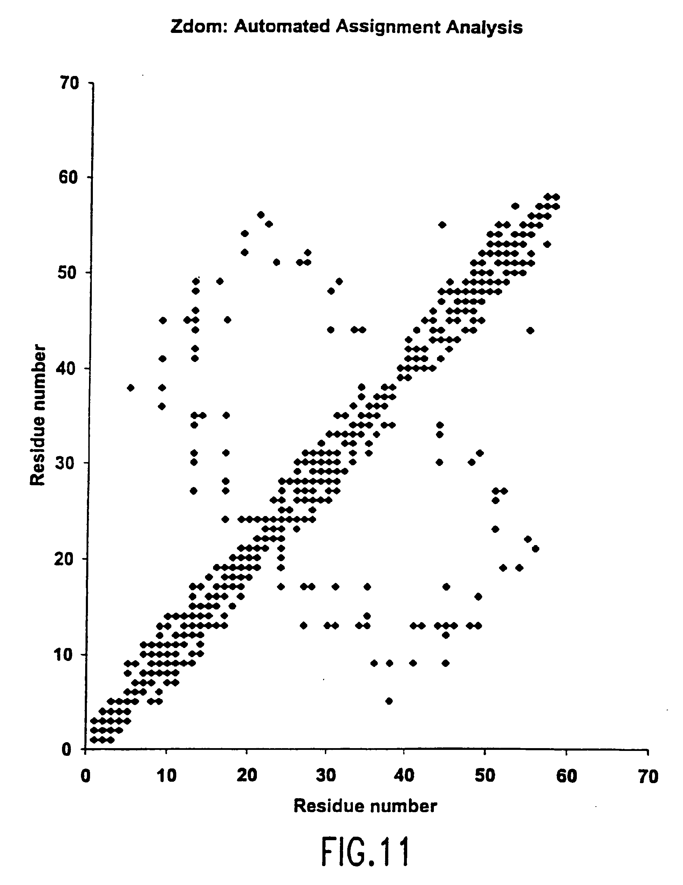

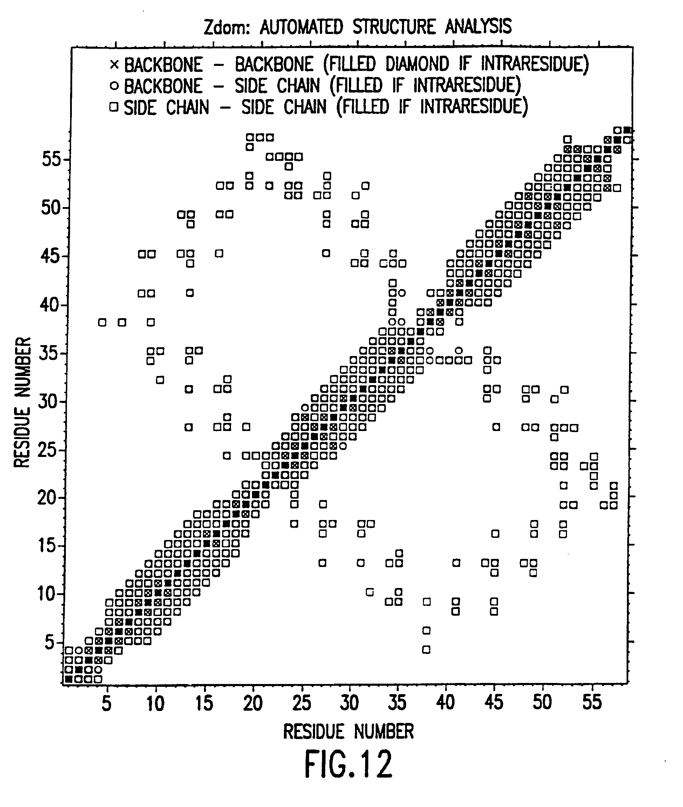

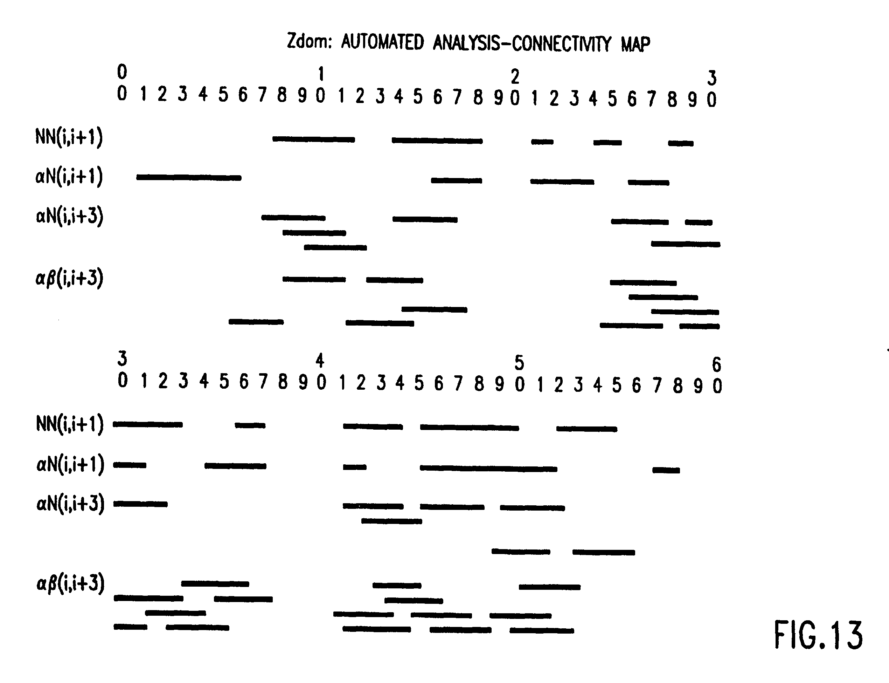

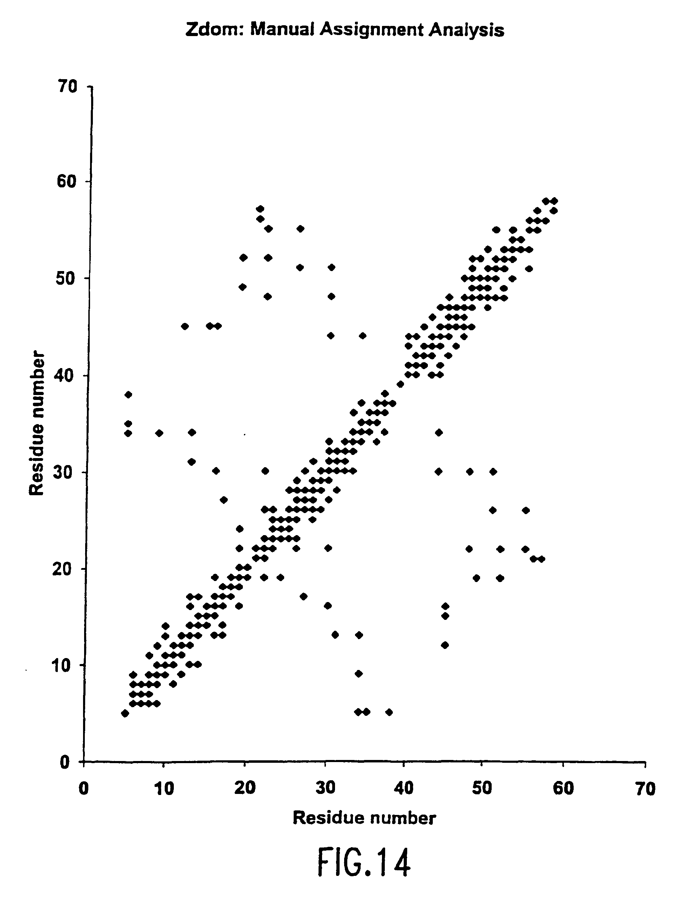

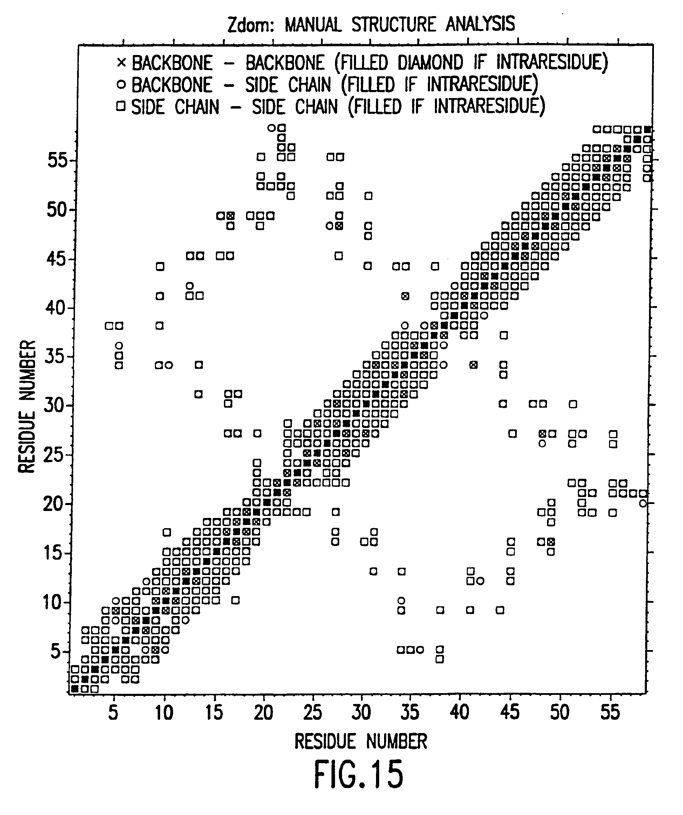

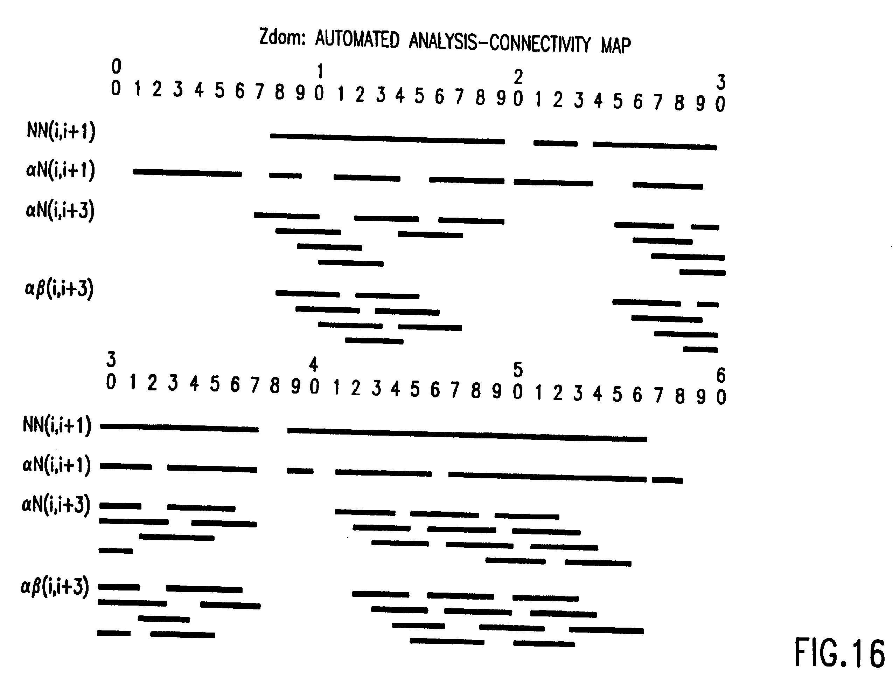

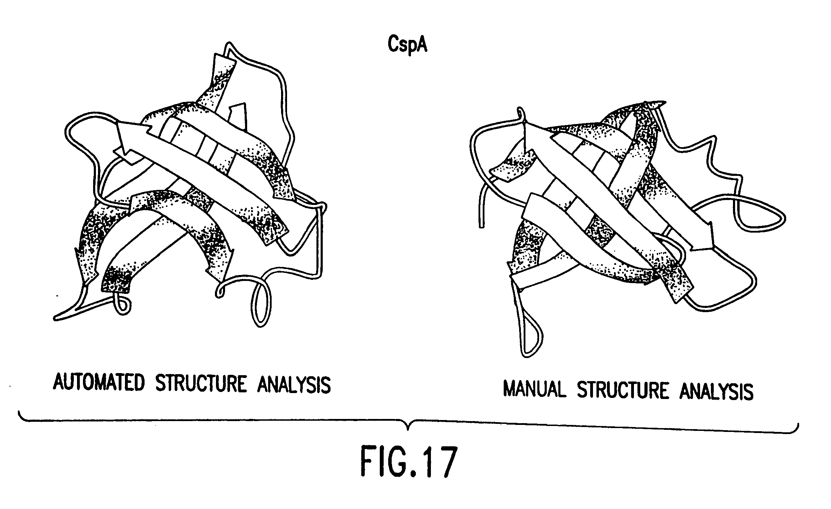

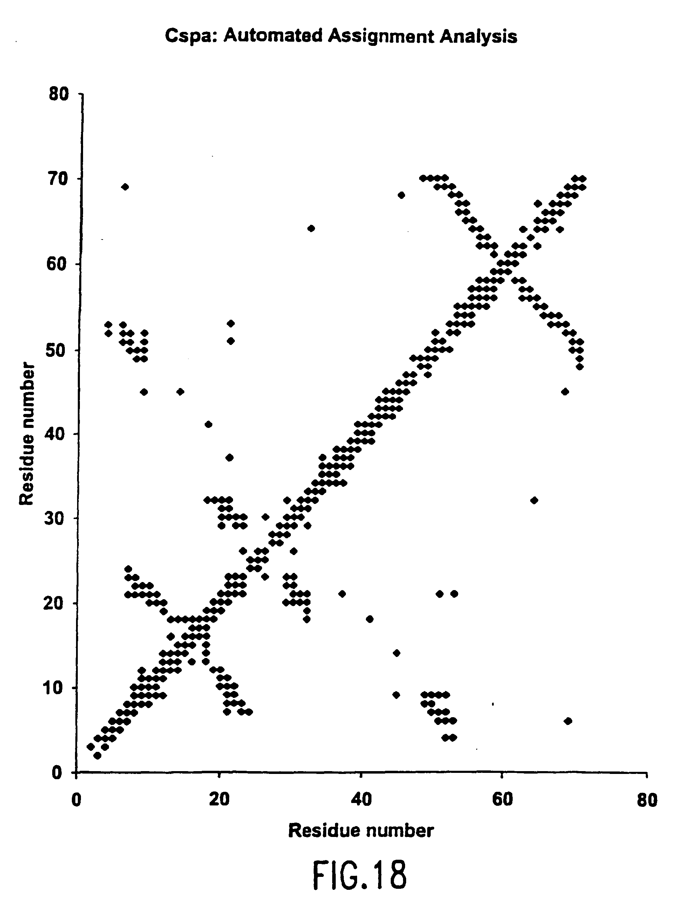

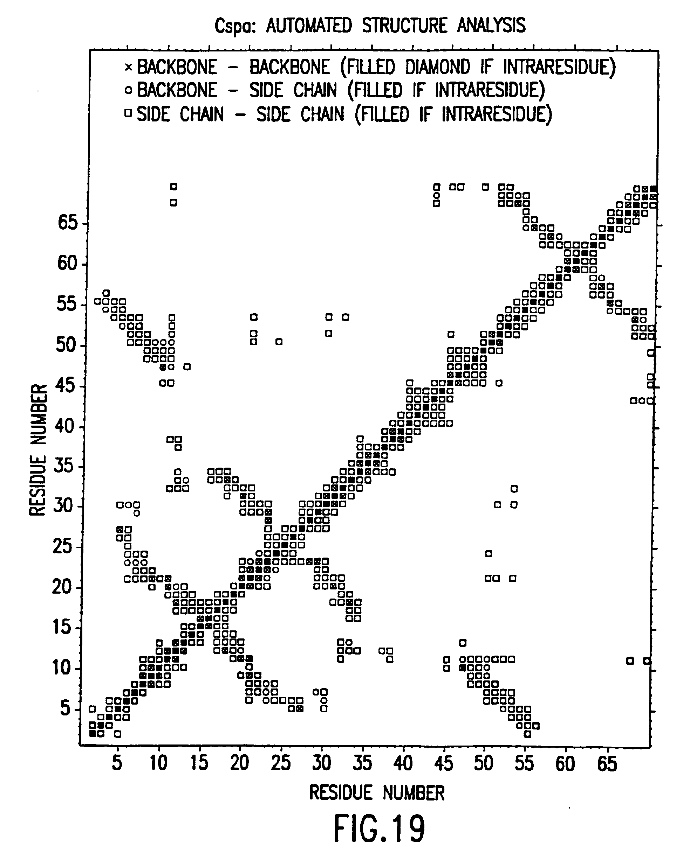

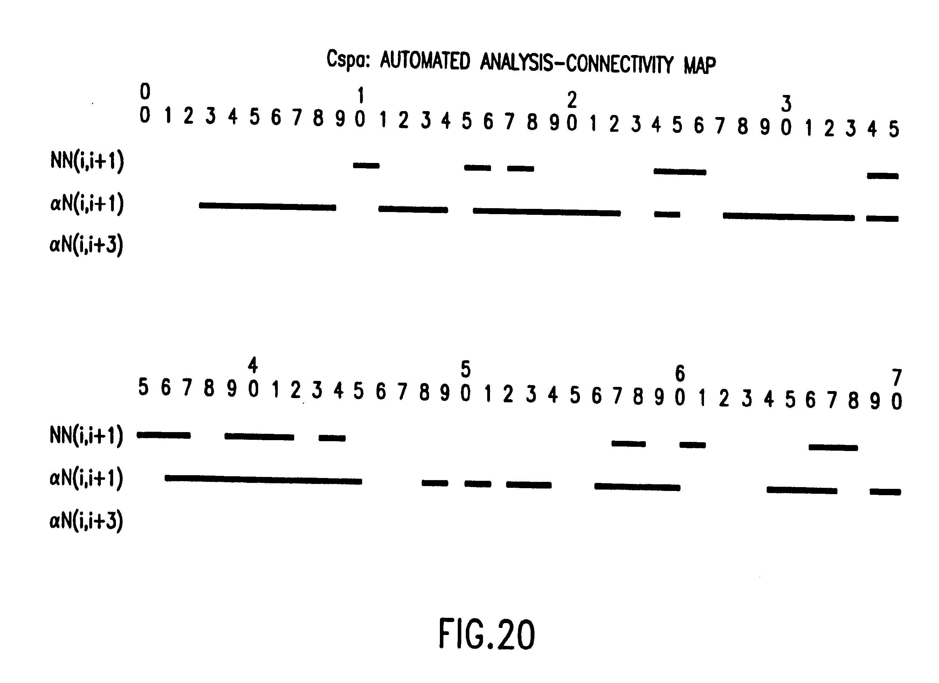

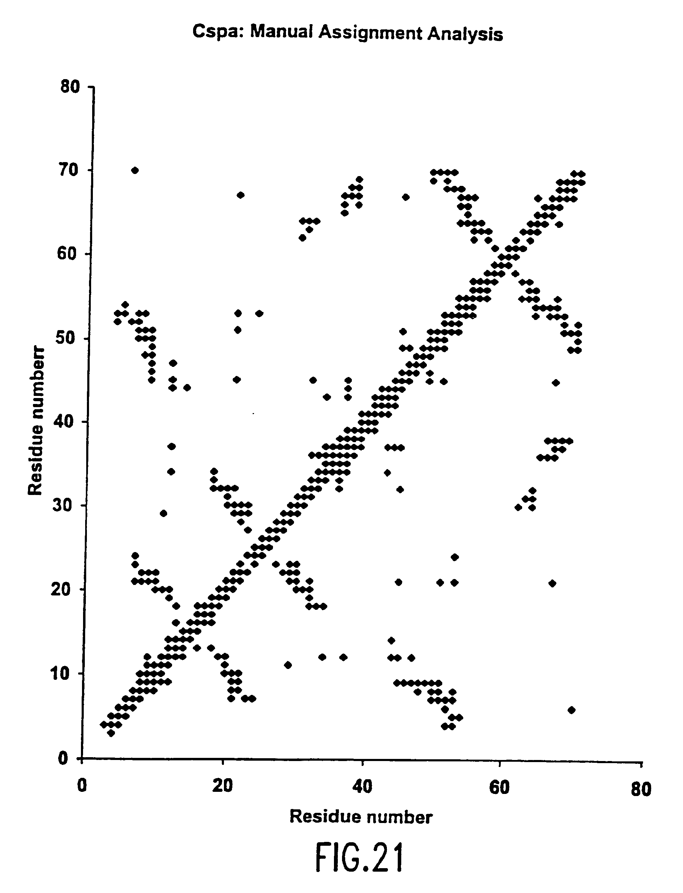

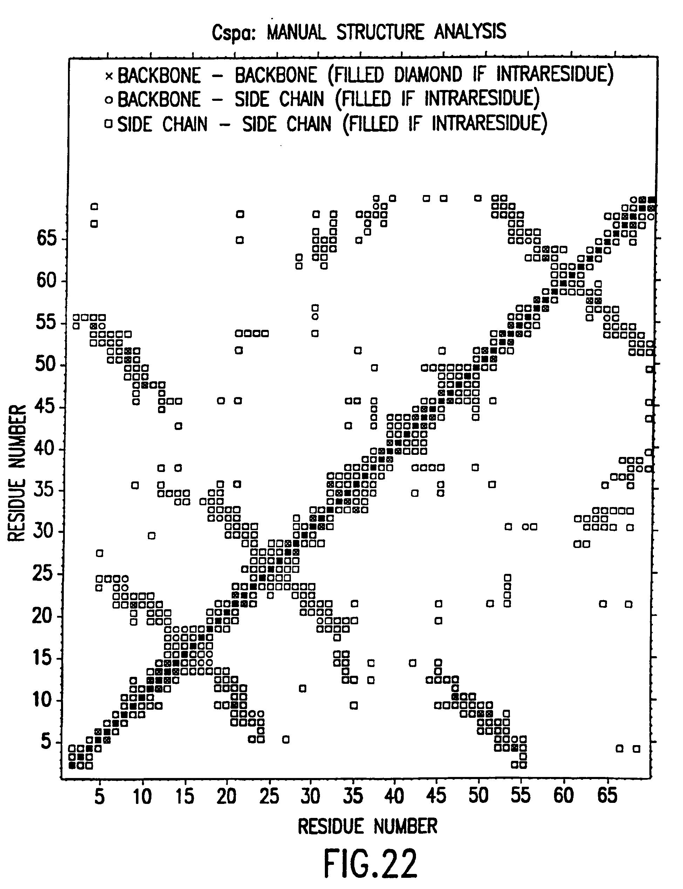

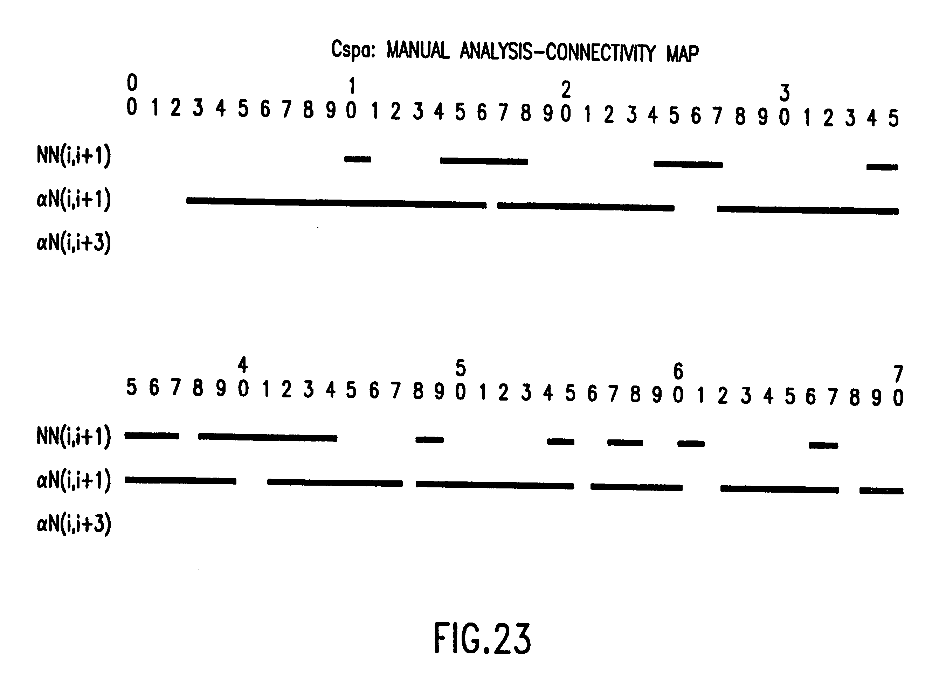

The constraint reasoning system, outlined in Figure 9 and described in Example 7. is used to automatically generate the 3D structures of the Zdom and Cspa proteins (Figures 10A and B, and Figure 17. respectively). The constraint reasoning system generated 3D structures are compared to the manually generated 3D structures. The results of the automated assignment analysis for Zdom and Cspa are presented in Figures 11-13 and 18-20, respectively. The results of the manual assignment analysis for Zdom and Cspa are presented in Figures 14-16 and 21-23, respectively. Backbone - backbone assignments are designated by x. Backbone - side chain assignments are designated by o. Side chain - side chain assignments are designated by o. Intra-residue assignments are designated by filled symbols.

In a further embodiment, a constraint reasoning system for automatically generating protein structures from NMR data is employed. A variety of constraints have been used to resolve the ambiguity problem in analysis of 2D and 3D NOESY spectra, obtain an initial chain fold, and then use constraints implied by this initial structure to iteratively refine the protein structure. The constraint reasoning system is based on automated analysis of secondary structure, prediction of hydrophobic core contacts, and iterative analysis of contact frequencies. The constraint reasoning system can generate reliable initial chain folds even when the chemical shift information alone provides few unambiguous NOESY cross peak assignments. Experimental NMR data for two different proteins have been analyzed to automatically generate 3D structures. The structures generated by this constraint reasoning system in hours are in good agreement with those derived from manual analysis processes which require weeks or months.

The NOESY-Assign constraint reasoning system for this purpose comprises the following 12 steps:

Step 1: Simple Match - get all possible assignments (A-type matches) for each spectra.

Step 2: Identify expected peaks which are intra/seq, or consistent with secondary structure. Put in U and E set. Create dynamically referenced values (DLRV) for H and HX (local referencing).

The DLRV for eacjh atom in each dimension includes the original chemical shift value plus any additional chemical shift values derived from E set.

Given a peak, if only one expected match is found, put in U and E set.

If found more than one expected match is found, select B-type expected matches, put in U and E set. See Step 5 for explanation of B-type match.

* Not for 2D spectra

- All assignments are D-type assignments
- Remove all that arc inconsistent with secondary structure

*** Possible features ***

1. Check if the data set are consistent with each other

- List the residue that no intra HN - Ha in N 15-NOESY
- List the residue that no intra Ha - Hb in C 13-NOESY
  If have, let the user do local re-refercing or global re-referencing

2. Do referencing refinement

Step 3: Define local match tolerance for HX dimension based on assigned HX resonance from E set.

* Not for 2D spectra
Define local match tolerance for HX dimension:

For each possible HX dimension assignment, find all assignments in E set, calculate the 60% confidence region. If the peak's chemical shift in the HX dimension is outside of the 60%

confident region (using common sample statistics methods), remove it from the list of possible assignments (flag).

Step 4: Supplement U based on chemical shift (unambiguous) with noise filter.
Noise filter: Basic idea is that real peaks have:

•intensity > 0.05% of the highest intensity of real peaks
•tighter match tolerance to chemical shift list than used in Step I (Simple Match)

Highest intensity of real peaks - what is real peaks? Use the highest intensity of intra peaks.
T-type assignments

Step 5: Define B-type matches, subset of A-type matches for each spectra.
The B-type matches arc defined as follows (by default):
For a given peak, order the A-type matches based on the size of the match value (MV), which is computed as follows:

$$MV = \min(\Delta HX + \Delta X/10 + \Delta H)$$

where:

$$\Delta H = H_{obs} - H_{DCSL}$$

$$\Delta HX = HX_{obs} - HX_{DCSL}$$

$$\Delta X = X_{obs} - X_{DCSL}$$

and $H_{DCSL}$, $HX_{DCSL}$, and $X_{DCSL}$ are the sets of dynamically locally referenced values (DLRV) for the H. HX and X dimensions, respectively. Choose all possible matches with: $\gamma \leq 0.01$, where $\gamma = |MV - (\Delta HX + \Delta X/10 + \Delta H|$.

Step 6: Use Contact Frequency (CF) of E to assign B-type matches to U set

* Not tor 2D spectra

•   Create contact bin from all E's
•   If element in B is in contact bin. Assign to U
•   T-type assignment

Step 7: Use SYM (Symmetry Property) to assign B-type matches to U

\* Not for 2D spectra
If peak in B has another symmetry peak in B, Assign both to U, as T-type assignments

Step 8:     Use HP-CORE to assign B-type matches to U

\* Not for 2D spectra
HP-CORE: Predicted Buried Residue

- Create HP-CORE contact bin from all B's

  - HP-CORE to HP-CORE
  - not in the same secondary segment

- If CF of the HP-CORE contact bin > N, assign all peaks in this bin to U. as T-type assignments. N is heuristic value that should scale with the number of NOESY spectra available, a typical value of N is 2.
- If element in B is in contact bin, Assign to U
- T-type assignment

Step 9:     Compute 3D structure

O: Order Parameter
0.8 (cycle 1). 0.7 (cycle 2), 0.6, (cycle 3), 0.5 (cycle 4)
1. Calibration

- D-type: distance is derived from its intensity
- T-type: distance = 5.0 Å

2. Run Structure Calculation Software
3. Select 10 best, from family of 50 3D structures:

- Compute: $S(\phi)$, $S(\varphi)$, $\sigma(i,j)$ matrix, bb rmsd (exclude record with $S(\phi) < 0.7$ and $S(\varphi) < 0.7$)
- if bb rmsd is too large. STOP
- if bb rmsd is < 1 Å, go to step 12

4. If has progress, go to step 10
5. If no more progress, decrease O (next cycle). Identify disordered regions - order(i,j), from O
If $(<S - O>>= 0$ & $\sigma(i,j) - 2/O <= 0$
Order (i,j) = 1 (ordered)
Else Order (i,j) = 0 (disordered)

Step 11:    Identify expected peaks that are consistent with 3D structure, put in U set.

D: Distance Cutoff
Assumption: If in ordered region, and > D, for sure, that is impossible to be a right assignment
If (order(i,j) = 1)

$$D = 5 + rmsd * 2 \text{ and } D_{min} = 5.5 \text{ Å}$$

N: Number of possible assignments left and put in U.
If rmsd > 2, N<=2, If rmsd > 1, N<=3,
Rest, N <=4,
Pruning A list:

- Remove possible assignement with dmn(i,j) > D in ordered region
- If N possible assignment left, put in U as T-type assignments

Step 12: Back calculate all possible NOE' that are expected from the structure. Output any predicted assignments not in U or A list and peaks still in A list.

Output:

For each cycle: Contact Map (assignment, structure(, Connectivity Map, Structures, Assignments (ordered by intra, seq, mid, long range), S($\phi$), S($\varphi$), σ(i,j) matrix, bb rmsd

Overview:

- Number of Assignments for each assignment step
- Table #Total NOE #U(D+T) #A #Noise
- Noise Peak List
- A-type matches List

**Claims**

1. A high-throughput method for determining a biochemical function of a protein or polypeptide domain of unknown function comprising:

   (A) identifying a putative polypeptide domain, 50 to 300 amino acids in length, that properly folds into a stable polypeptide domain, said stable polypeptide having a defined three dimensional structure;
   (B) determining the three dimensional structure of the stable polypeptide domain from an automated analysis of NMR spectometer spectra of said polypeptide domain, wherein said automated analysis is conducted by a NOESY_Assign process;
   (C) comparing the determined three dimensional structure of the stable polypeptide domain to known three-dimensional structures in a protein data bank, wherein said comparison identifies known structures within said protein data bank that are homologous to the determined three dimensional structure; and
   (D) correlating a biochemical function corresponding to the identified homologous structure to a biochemical function for the stable polypeptide domain.

2. A method according to claim 1, further comprising the prestep of parsing a target polynucleotide into at least one putative polypeptide domain.

3. A method according to claim 2, wherein said parsing is performed by a first computer algorithm, wherein said first computer algorithm is selected from a computer algorithm capable of determining exon phase boundaries of a polynucleotide, and a computer algorithm capable of determining interdomain boundaries encoded in a polynucleotide.

4. A method according to claim 3, further comprising a computer algorithm that compares the putative polypeptide domain sequence with known domain sequences stored within a database.

5. A method according to any preceding claim wherein said NMR spectra are analyzed by a second computer algorithm that automatically assigns resonance assignments to the polypeptide sequence.

6. A method according to any preceding claim, wherein said identification of the said stable polypeptide domain comprises measuring a time course of amide hydrogen-deuterium exchange.

7. A method according to any preceding claim, wherein prior to step (B), said stable polypeptide domain is optimally solubilized, said optimum solubilization comprising:

   i) preparing an array of microdialysis buttons, wherein each of the said microdialysis buttons contains at least substantially 1 $\mu$l of a substantially 1mM solution of said stable polypeptide domain;
   ii) dialyzing each member of said array of microdialysis buttons against a different dialysis buffer;

iii) analyzing each of said dialyzed microdialysis buttons to determine whether said stable polypeptide domain has remained soluble; and
iv) selecting the polypeptide domain having optimum solubility characteristics for NMR spectroscopy.

**8.** A method according to any preceding claim wherein said comparison of said determined three dimensional structure to said known three-dimensional structures in the protein data bank is performed by a third computer algorithm that is capable of determining 3D structure homology between said determined three dimensional structures and a member of said protein data bank.

**9.** A method according to claim 8, wherein said third computer algorithm is selected from DALI, CATH and VAST.

**10.** A method according to any preceding claim, wherein said protein data bank is Protein Data Base ("PDB").

**11.** A high-throughput method for determining a biochemical function of a polypeptide of unknown function encoded by a target polynucleotide, comprising the steps:

(A) identifying at least one putative polypeptide domain encoding region of the target polynucleotide ("parsing");
(B) expressing said putative polypeptide domain;
(C) determining whether said expressed putative polypeptide domain forms a stable polypeptide domain having a defined three dimensional structure ("trapping");
(D) determining the three dimensional structure of the stable polypeptide domain by an automated NOESY_ Assign process;
(E) comparing the determined three dimensional structure of the stable polypeptide domain to known three-dimensional structures of a protein data bank to determine whether any such known structures are homologous to the determined structure; and
(F) correlating a biochemical function corresponding to the homologous structure to a biochemical function for the stable polypeptide domain.

**Patentansprüche**

**1.** Verfahren mit hohem Durchsatz zum Bestimmen einer biochemischen Funktion eines Protein- oder Polypeptid-Bereichs einer unbekannten Funktion, umfassend:

(A) Identifizieren eines putativen Polypeptid-Bereichs von 50 bis 300 Aminosäuren in der Länge, welcher sich passend in einen stabilen Polypeptid-Bereich faltet, wobei das stabile Polypeptid eine definierte dreidimensionale Struktur aufweist;
(B) Bestimmen der dreidimensionalen Struktur des stabilen Polypeptid-Bereichs aus einer automatisierten Analyse von NMR-Spektrometer-Spektren des Polypeptid-Bereichs, wobei die automatisierte Analyse durch einen NOESY_Assign-Prozess durchgeführt wird;
(C) Vergleichen der bestimmten dreidimensionalen Struktur des stabilen Polypeptid-Bereichs mit bekannten dreidimensionalen Strukturen in einer Protein-Datenbank, wobei der Vergleich bekannte Strukturen innerhalb der Protein-Datenbank identifiziert, die homolog zu der bestimmten dreidimensionalen Struktur sind; und
(D) Korrelieren einer biochemischen Funktion, die der identifizierten homologen Struktur entspricht, auf eine biochemische Funktion für den stabilen Polypeptid-Bereich.

**2.** Verfahren gemäß Anspruch 1, weiter umfassend den vorangestellten Schritt eines Parsing eines Ziel-Polynukleotids in mindestens einen putativen Polypeptid-Bereich.

**3.** Verfahren gemäß Anspruch 2, wobei das Parsing durch einen ersten Computer-Algorithmus durchgeführt wird, wobei der erste Computer-Algorithmus aus einem Computer-Algorithmus gewählt wird, welcher es erlaubt, Exon-Phasengrenze eines Polynukleotids zu bestimmen, und einem Computer-Algorithmus, welcher es erlaubt, Zwischenbereichsgrenzen zu bestimmen, die in einem Polynukleotid kodiert sind.

**4.** Verfahren gemäß Anspruch 3 weiter umfassend einen Computer-Algorithmus, der die putative Polypeptid-Bereichssequenz mit bekannten Bereichssequenzen vergleicht, die in einer Datenbank gespeichert sind.

**5.** Verfahren gemäß irgendeinem der vorhergehenden Ansprüche, wobei die NMR-Spektren durch einen zweiten Computer-Algorithmus analysiert werden, der automatisch Resonanzzuordnungen der PolypeptidSequenz zuordnet.

6. Verfahren gemäß irgendeinem der vorhergehenden Ansprüche, wobei die Identifikation des stabilen Polypeptid-Bereichs ein Messen eines Zeitverlaufs eines Amid-Wasserstoff-Deuterium-Austausches umfasst.

7. Verfahren gemäß irgendeinem der vorhergehenden Ansprüche, wobei vor dem Schritt (B) der stabile Polypeptid-Bereich optimal aufgelöst ist, wobei die optimale Auflösung umfasst:

> i) Vorbereiten eines Arrays von Mikrodialyseknöpfen, wobei jeder der Mikrodialyseknöpfe im Wesentlichen mindestens 1 µl einer im Wesentlichen 1mM-Lösung des stabilen Polypetid-Bereichs enthält;
> ii) Dialysieren jedes Elements des Arrays von Mikrodialyseknöpfen gegenüber einem unterschiedlichen Dialysepuffer;
> iii) Analysieren von jedem der dialysierten Mikrodialyseknöpfe um zu bestimmen, ob der stabile Polypeptid-Bereich auflösbar geblieben ist; und
> iv) Auswählen des Polypeptid-Bereichs, welcher optimale Löslichkeitscharakteristiken für eine NMR-Spektroskopie aufweist.

8. Verfahren gemäß irgendeinem der vorhergehenden Ansprüche, wobei der Vergleich der bestimmten dreidimensionalen Struktur mit bekannten dreidimensionalen Strukturen in der Proteindatenbank mittels eines dritten Computer-Algorithmus durchgeführt wird, welcher ein Bestimmen einer 3D-Struktur-Homologie zwischen den bestimmten dreidimensionalen Strukturen und einem Element der Proteindatenbank ermöglicht.

9. Verfahren gemäß Anspruch 8, wobei der dritte Computer-Algorithmus gewählt wird aus DALI, CATH und VAST.

10. Verfahren gemäß einem der vorhergehenden Ansprüche, wobei die Proteindatenbank eine Proteindatenbasis ("PDB") ist.

11. Verfahren mit hohem Durchsatz zum Bestimmen einer biochemischen Funktion eines Polypeptids einer unbekannten Funktion, welches durch ein Ziel-Polynukleotid kodiert ist, umfassend die Schritte:

> (A) Identifizieren mindestens einer putativen Polypetid-Bereichs-Kodierungsregion des Ziel-Polynukleotids ("Parsing");
> (B) Exprimieren des putativen Polypeptid-Bereichs;
> (C) Bestimmen, ob der exprimierte putative Polypeptid-Bereich einen stabilen Polypeptid-Bereich bildet, welcher eine definierte dreidimensionale Struktur ("Trapping") aufweist;
> (D) Bestimmen der dreidimensionalen Struktur des stabilen Polypeptid-Bereichs durch einen automatisierten NOESY_Assign-Prozess;
> (E) Vergleichen der bestimmten dreidimensionalen Struktur des stabilen Polypeptid-Bereichs mit bekannten dreidimensionalen Strukturen einer Proteindatenbank, um zu bestimmen, ob irgendwelche derartigen bekannten Strukturen homolog zu der bestimmten Struktur sind;
> (F) Korrelieren einer biochemischen Funktion, die der homologen Struktur entspricht, auf eine biochemische Funktion für den stabilen Polypeptid-Bereich.

**Revendications**

1. Procédé à haut débit pour déterminer une fonction biochimique d'un domaine de protéine ou de polypeptide de fonction inconnue, comprenant les opérations consistant à :

> (A) identifier un domaine de polypeptide putatif, de 50 à 300 acides aminés de longueur, qui se replie de façon appropriée en un domaine de polypeptide stable, ledit polypeptide stable ayant une structure tridimensionnelle définie ;
> (B) déterminer la structure tridimensionnelle du domaine de polypeptide stable à partir d'une analyse automatisée de spectres de spectromètre RMN dudit domaine de polypeptide, ladite analyse automatisée étant conduite par un processus NOESY_Assign ;
> (C) comparer la structure tridimensionnelle déterminée du domaine de polypeptide stable à des structures tridimensionnelles connues dans une banque de données de protéines, ladite comparaison identifiant des structures connues à l'intérieur de ladite banque de données de protéines qui sont homologues à la structure tridimensionnelle déterminée ; et
(D) corréler une fonction biochimique correspondant à la structure homologue identifiée à une fonction biochimique pour le domaine de polypeptide stable.

**2.** Procédé selon la revendication 1, comprenant en outre la pré-étape de segmentation d'un polynucléotide cible en au moins un domaine de polypeptide putatif.

**3.** Procédé selon la revendication 2, dans lequel ladite segmentation est effectuée par un premier algorithme d'ordinateur, ledit premier algorithme d'ordinateur étant choisi parmi un algorithme d'ordinateur capable de déterminer des limites de phases d'exons d'un polynucléotide, et un algorithme d'ordinateur capable de déterminer des limites interdomaines codées dans un polynucléotide.

**4.** Procédé selon la revendication 3, comprenant en outre un algorithme d'ordinateur qui compare la séquence de domaine de polypeptide putatif avec des séquences de domaines connues stockées dans une base de données.

**5.** Procédé selon l'une quelconque des revendications précédentes, dans lequel lesdits spectres de RMN sont analysés par un second algorithme d'ordinateur qui attribue automatiquement des attributions de résonance à la séquence polypeptidique.

**6.** Procédé selon l'une quelconque des revendications précédentes, dans lequel ladite identification dudit domaine de polypeptide stable comprend la mesure d'une évolution d'échange hydrogène-deutérium d'amide.

**7.** Procédé selon l'une quelconque des revendications précédentes, dans lequel avant l'étape (B), ledit domaine de polypeptide stable est solubilisé de façon optimale, ladite solubilisation optimale comprenant les opérations consistant à :

(i) préparer un ensemble de boutons de microdialyse, chacun desdits boutons de microdialyse contenant au moins sensiblement 1 µl d'une solution sensiblement 1 mM dudit domaine de polypeptide stable ;
(ii) dialyser chaque élément dudit ensemble de boutons de microdialyse contre un tampon de dialyse différent ;
(iii) analyser chacun desdits boutons de microdialyse dialysés pour déterminer si ou non ledit domaine de polypeptide stable est resté soluble ; et
(iv) sélectionner le domaine de polypeptide ayant des caractéristiques de solubilité optimale pour une spectroscopie RMN.

**8.** Procédé selon l'une quelconque des revendications précédentes, dans lequel ladite comparaison de ladite structure tridimensionnelle déterminée auxdites structures tridimensionnelles connues dans la banque de données de protéines est effectuée par un troisième algorithme d'ordinateur qui est capable de déterminer une homologie de structure tridimensionnelle entre lesdites structures tridimensionnelles déterminées et un élément desdites banques de données de protéines.

**9.** Procédé selon la revendication 8, dans lequel ledit troisième algorithme d'ordinateur est choisi parmi DALI, CATH et VAST.

**10.** Procédé selon l'une quelconque des revendications précédentes, dans lequel ladite banque de données de protéines est la Base de Données de Protéines (« Protein Data Base - PDB »).

**11.** Procédé à haut débit pour déterminer une fonction biochimique d'un polypeptide de fonction inconnue codé par un polynucléotide cible, comprenant les étapes consistant à :

(A) identifier au moins une région du polynucléotide cible codant pour un domaine de polypeptide putatif (« segmentation ou parsing ») ;
(B) exprimer ledit domaine de polypeptide putatif ;
(C) déterminer si ou non ledit domaine de polypeptide putatif exprimé forme un domaine de polypeptide stable ayant une structure tridimensionnelle définie (« piégeage ou trapping ») ;
(D) déterminer la structure tridimensionnelle du domaine de polypeptide stable par un processus NOESY_ Assign automatisé ;
(E) comparer la structure tridimensionnelle déterminée du domaine de polypeptide stable pour connaître des structures tridimensionnelles d'une banque de données de protéines afin de déterminer si ou non n'importe quelles de ces structures connues sont homologues à la structure déterminée ; et

(F) corréler une fonction biochimique correspondant à la structure homologue à une fonction biochimique pour le domaine de polypeptide stable.

FIG.1

FIG.2A

FIG.2B

FIG.3

FIG.4

FIG.5

25°C
NTD 2-3 CLEARED – 8HR $^{15}$NHSQC

$^{15}$N (ppm)

$^{1}$H (ppm)

FIG.6

EP 1 104 488 B1

FIG.7

5 10 15 20 25 30 35 40 45 50 55 60 65 70

MSGKMTGIVKWFNADKGFGFITPDDGSKDVFVHFSAIQNDGYKSLDEGQKVSFTIESGAKGPAAGNVTSL

Cα

Cβ

Cγ

Cδ

Cε

Hα

Hβ

Hγ

Hδ

Hε

FIG.8A

EP 1 104 488 B1

FIG.8B

NOESY_ASSIGN PROCESS

STEP 1: SIMPLE MATCH-GET ALL POSSIBLE ASSIGNMENT (A-TYPE MATCH).

STEP 2: IDENTIFY EXPECTED PEAKS WHICH ARE INTRA/SEQ, OR CONSISTENT WITH SECONDARY STRUCTURE. PUT IN U AND E SET. CREATE DLRV.

STEP 3: DEFINE LOCAL MATCH TOLERANCE FOR HX DIMENSION BASED ON ASSIGNED HX RESONANCES FROM E SET.

STEP 4: SUPPLEMENT U BASED ON CHEMICAL SHIFT (UNAMBIGUOUS) WITH NOISE FILTER.

STEP 5: DEFINE B-TYPE MATCHES OF A-TYPE MATCHES.

STEP 6: USE CF OF E TO ASSIGN B TO U.

STEP 7: USE SYM TO ASSIGN B TO U.

STEP 8: USE HP-CORE TO ASSIGN B TO U.

STEP 9: COMPUTE 3D STRUCTURE.

STEP 10: VALIDATION-REMOVE FROM U LIST THE CONSISTENTLY VIOLATED NOE ASSIGNMENTS.

STEP 11: IDENTIFY EXPECTED PEAKS THAT ARE CONSISTENT WITH 3D STRUCTURE, PUT IN U SET.

STEP 12: BACK CALCULATE ALL POSSIBLE NOE'S THAT ARE EXPECTED FROM STRUCTURE. OUTPUT ANY PREDICTED ASSIGNMENTS NOT IN U OR A LIST AND PEAKS STILL IN THE A LIST.

FIG.9

FIG.10A

Automated Structure Analysis          Manual Structure Analysis

FIG.10B

**Zdom: Automated Assignment Analysis**

FIG.11

FIG.12

Zdom: AUTOMATED ANALYSIS—CONNECTIVITY MAP

FIG.13

Zdom: Manual Assignment Analysis

FIG.14

FIG.15

Zdom: AUTOMATED ANALYSIS-CONNECTIVITY MAP

FIG.16

CspA

AUTOMATED STRUCTURE ANALYSIS          MANUAL STRUCTURE ANALYSIS

FIG.17

Cspa: Automated Assignment Analysis

FIG.18

Cspa: AUTOMATED STRUCTURE ANALYSIS

FIG.19

Cspa: AUTOMATED ANALYSIS-CONNECTIVITY MAP

FIG.20

Cspa: Manual Assignment Analysis

FIG.21

Cspa: MANUAL STRUCTURE ANALYSIS

FIG.22

Cspa: MANUAL ANALYSIS–CONNECTIVITY MAP

FIG.23